# EUROPEAN PATENT SPECIFICATION

(11) **EP 1 620 719 B1**
(45) Date of publication and mention of the grant of the patent: **17.09.2008**
(21) Application number: 04731042.0
(22) Date of filing: 04.05.2004
(51) Int. Cl.: G01N 33/00

(54) **BIOMOLECULAR SWITCHING DEVICE**
BIOMOLEKULARE SCHALTVORRICHTUNG
DISPOSITIFS BIOMOLECULAIRES

(30) Priority: 03.05.2003 GB 0310270
(43) Date of publication of application: 01.02.2006
(73) Proprietor: The University Court of the University of Edinburgh, Edinburgh EH8 9YL (GB)
(72) Inventor: CRAIN, Jason, Edinburgh EH21 6QC (GB); WALTON, Anthony, John, Edinburgh EH10 7AT (GB); GHAZAL, Peter, Edinburgh EH16 5QW (GB); MOUNT, Andrew, Raymond, Edinburgh EH16 6GJ (GB); BEATTIE, John, Samuel, Edinburgh EH9 2PH (GB); CAMPBELL, Colin, James, Midlothian EH25 9 LP (GB); TERRY, Jonathan, Gordon, Durham DH1 3QP (GB); EVANS, Stuart, Anthony, Grant, Clanfield, Bampton, Oxforshire OX18 2SJ (GB)
(74) Representative: Szczuka, Jan Tymoteusz
(86) International application number: PCT/GB2004/001947
(87) International publication number: WO 2004/099767

(56) References cited:
- WO-A-02/095840
- GB-A- 2 266 182
- MCKINNEY S A ET AL: "Structural dynamics of individual Holliday junctions" NATURE STRUCTURAL BIOLOGY, vol. 10, no. 2, 23 December 2002 (2002-12-23), pages 93-97, XP002294545 USA ISSN: 1072-8368
- REIF J H ET AL: "CHALLENGES AND APPLICATIONS FOR SELF-ASSEMBLED DNA NANOSTRUCTURES" LECTURE NOTES IN COMPUTER SCIENCE, SPRINGER VERLAG, NEW YORK, NY, US, vol. 2054, 13 June 2000 (2000-06-13), pages 173-198, XP008006848 ISSN: 0302-9743
- WILLNER I ET AL: "PHOTOSWITCHABLE BIOMATERIALS AS GROUNDS FOR OPTOBIOELECTRONIC DEVICES" BIOELECTROCHEMISTRY AND BIOENERGETICS, vol. 42, no. 1, April 1997 (1997-04), pages 43-57, XP002039173 ISSN: 0302-4598
- KUWABARA TOMOKO ET AL: "Allosterically controllable ribozymes with biosensor functions" CURRENT OPINION IN CHEMICAL BIOLOGY, vol. 4, no. 6, December 2000 (2000-12), pages 669-677, XP002348297 ISSN: 1367-5931

## Description

The present invention relates to macromolecular switches .

There is a need for systems capable of probing target biomolecules and target biomolecule interactions. This requires not only that target biomolecule binding and target biomolecule interactions occur with high specificity, but also that these should be reliably and efficiently detected and processed.

Various, essentially biochemical, techniques are known in the art which generally involve binding suitably labelled biomolecules, with greater or lesser affinity, to a target biomolecule so as to form a labelled conjugate, discarding unbound material, and then measuring the amount of labelled material present by suitable techniques. One limitation of such processes is that they involve significant handling of sample material and probing material to remove all unbound labelled material in order to obtain reliable measurements of labelled conjugate. A significant further problem is that capturing and processing the data obtained from such measurements is generally very disjointed and cumbersome. Thus there is a need for more reliable, sensitive and versatile systems for signalling out target biomolecule binding and other interactions.

Whilst in the past, the desirability of such better systems has been generally recognized, the various specific proposals that have been made, have suffered from a number of shortcomings and disadvantages.

GB2266182 discloses in very general terms, the concept of biomolecular switches based on a wide range of different biomolecules which can exist in two or more states of comparable stability, with a very wide range of different kinds of state including differences in (any) chemical or physical property, quantum mechanical electronic spin state, biological activity, and three-dimensional conformation. There is however, very little guidance to the reader as to how such a concept could actually be realised, and the disclosure leads off in many different directions which are impractical and/or problematic. Thus for example the only state switching mentioned in relation to nucleic acids is that involving melting of ds DNA resulting in disintegration of the macromolecular structure followed by annealing, which would be extremely unlikely to result in recombination of the particular strands forming the original individual macromolecular structure. Moreover the only concrete proposal relates to "switching" of an array of riboflavin binding proteins (RBPs) between one "state" in which they are bound to a riboflavin ligand and another "state" in which they are not bound to a riboflavin ligand, said switching being controlled by interconnection of the individual macromolecules in the array through a gradient pattern of stimuli caused by change in pH. Whilst this is referred to as a switching of "states", it actually corresponds to a switching between different macromolecular entities involving complete disintegration of the RBP-riboflavin complex and formation of new individual complex entities. This is quite different from switching between well defined states of one and the same macromolecular structure.

It is an object of the present invention to avoid or minimise one or more of the above problems or disadvantages of the prior art.

A key object of the invention is to provide a macromolecular structure switch suitable for integration with electronic circuitry and interacting with biomolecules, whereby processing of data relating to biomolecule interactions may be facilitated.

In one aspect the present invention provides:
a macromolecular switch
suitable for use in a data acquisition and/or processing device, comprising:
   a macromolecular structure
   having at least one ion binding site, and separatively at least one biomolecule analyte binding site formed and arranged for binding to a predetermined target biomolecule analyte, said macromolecular structure being flippable
   between a plurality of discretely different conformations corresponding to different ion binding conditions at said at least one ion binding site,
   at least in the absence of any other binding to said macromolecular structure,
   said macromolecular structure being provided with at least one electrochemical input device formed and arranged for applying such different ion binding conditions to said ion binding site in response to corresponding external input signals.

It is important to note that the macromolecular structures used in accordance with the present invention are flippable between more or less well defined discretely different conformations or structural forms. This is quite different from previously known systems in which it has been proposed to use macromolecules which are described as being flippable between different "states", which typically correspond to one state in which the macromolecule is bound to some other moiety, and another state in which it is not bound to said moiety, and/or involve one or more conformational states which is (are) not well defined to a greater or lesser extent. The use of the particular type of flippable macromolecular structures according to the present invention, provides better defined and quantized or digitized switching characteristics with more clearly and positively defined switch states which leads to significant benefits such as more reliable and reproducible operation.

For the avoidance of doubt, it should be noted that the following terminology used herein in relation to various features of the present invention is intended to encompass the following meaning, unless otherwise specifically indicated.

The term "detecting" indicates qualitative and/or quantitative detection, including inter alia simply detecting the presence or absence of a target analyte, and simply detecting a change in the concentration of a target analyte.

The expression "ion binding conditions" includes both physico-chemical conditions such as concentration, ionic strength, temperature, electric field strength etc, and also features such as the nature, identity, ion size, etc of the particular ion species involved in binding to the ion binding site(s).

The macromolecular switches provided by the present invention have a wide range of possible applications, ranging from simple switches suitable for use in data processing circuitry, to more complex data signal capture and/or processing applications, which may or may not require conformation switching of the macromolecular structure in use thereof. Such more complex applications include detection of specific target biomolecule analytes (which may moreover include biomolecule analytes which form part of another macromolecular structure switch), through the detection of binding to high specificity biomolecule analyte binding sites. Such binding can be detected in conventional manner by detecting the presence of bound labelled material where a suitably labelled target analyte has been used. The switches of the present invention do however offer the possibility of significantly improved detection systems.

Thus in some preferred forms of the invention there may be used a multi-component separation sensitive output device having one output device component mounted directly on a first part of the macromolecular structure and a second output device component located on a target biomolecule analyte, said output device being formed and arranged so as to provide an output signal only when both said first and second output device components are present on the macromolecular structure i.e. when the target biomolecule labelled with said second output device, has been bound to the biomolecule analyte binding site. This can be used to provide improved reliability in analyte detection by providing a (positive) output signal only when target biomolecule analyte has been specifically bound to the biomolecule analyte binding site, and not in the case of nonspecific binding of biomolecule analyte elsewhere than to the macromolecular structure.

It is also possible to achieve biomolecule analyte detection with some preferred forms of the invention without the use of any target biomolecule analyte labelling, thereby considerably simplifying the processing required and broadening the scope of possible applications, by means of using a macromolecular structure with a target biomolecule analyte binding site with a competitor biomolecule bound thereto, which competitor biomolecule is substitutable by a said target biomolecule analyte in use of the switch, by means of competitive binding. The target biomolecule substitution may be detected in various ways, for example, by using a labelled competitor biomolecule, the displacement of the competitor biomolecule being detectable by the loss of label signal. Another possibility would be to use a competitor biomolecule which is bound to said macromolecular structure (for example binding across two arms of a macromolecular structure such as a Holliday Junction - further described hereinbelow and conveniently referred to herein as an HJ) so as to lock said structure in one of said conformations against flipping to a different said conformation, the displacement of the competitor biomolecule from the biomolecule binding site being detectable by the restoration of conformation flippability, by monitoring the results of application of a conformation flipping signal - generally via changing of the ion binding conditions.

The use of two different competitor biomolecules binding across different pairs of arms of an HJ so as to lock it against flipping affords the possibility of providing an AND gate device in which flipping could only occur in the presence of both of two different target biomolecule analytes displacing the competitor biomolecule from each of the two different biomolecule analyte binding sites. Similarly an HJ structure with two pairs of biomolecule binding sites for binding to respective portions of two different target biomolecule analytes, may be used as a NOR-Gate which is locked against flipping from an open conformation to a closed conformation, in the presence of either or both of said two different target biomolecule analytes, and can only be flipped from an open conformation to a closed conformation in the complete absence of either.

The use of HJ structures with pairs of biomolecule analyte binding sites for competitor biomolecule binding across pairs of arms of an HJ so as to lock it against flipping, also provides another form of memory device element, which is writable by means of contacting the HJ structure with such a competitor biomolecule - so as to lock it against flipping from an open conformation to a closed conformation, and erasable by contacting with a target biomolecule which displaces the competitor biomolecule from at least one of said pairs of biomolecule analyte binding sites.

A particular benefit of the combination of conformation switching and analyte binding, is that it enables temporal control of analyte binding and/or of reading out of analyte binding, in a particularly simple and convenient manner. Thus, where there is provided a macromolecular structure formed and arranged so as to selectively restrict analyte access to the biomolecule analyte binding site in one of said conformations so that biomolecule analyte binding can only take place in an (or the) other conformation, it is possible to control more or less precisely the timing of when a sample is probed for the presence of a target analyte, allowing, for example, the probing system incorporating said macromolecular structure to be brought into contact with the sample and then "switching on" the probing system by flipping its conformation from an inaccessible to an accessible biomolecule analyte binding site condition, at a particular desired time. Furthermore by providing a probing system in which a series of identical macromolecular structures is selectively "switched on" at a series of different times, it is possible to carry out temporal analysis and monitor change in biomolecule analytes over a period of time.

Temporal control of reading out of biomolecule analyte binding can be achieved where the biomolecule analyte incorporates an output device component of a separation sensitive output signal system which is only active in one of said conformations, and the macromolecular structure is held in another conformation, until it is desired to read out any biomolecule analyte binding, whereupon the macromolecular structure conformation is flipped to the "active" conformation.

Another benefit obtainable with macromolecular structures having multiple different biomolecule analyte binding sites, is that of facilitating more accurate stoichiometric analyses to be carried out. It will be appreciated that practical applications of the macromolecular structure switches of the invention will generally involve more or less large populations of the macromolecular structures (typically hundreds or thousands) whereby it will often be difficult to ensure that any given device contains exactly the same number of individual macromolecular structures as another device. By providing a number of binding sites for different biomolecule analytes on the same macromolecular structure, it can be ensured that whatever the number of macromolecular structures used in a device, the proportions of binding sites for different biomolecule analytes, will remain the same.

Another advantage of using macromolecular structures with multiple (different) biomolecule analyte binding sites, is that it can enable a degree of data processing integration, by reducing the signal processing required in relation to multiple analyte detection. Thus, for example, by using a separation sensitive signal transmission system as described above with respective output device components, each of which, however, is disposed on a respective different biomolecule analyte, an output signal will only be obtainable when both biomolecule analytes are present and bound to the macromolecular structure, thereby effectively providing a "parallel processing" of the detection of two different biomolecule analytes, thereby avoiding the need for processing of two separate signals corresponding to the detection of the different biomolecule analytes.

With regard to the provision of biomolecule analyte binding sites, in the case of HJ macromolecular structures, these can conveniently be in the form of so-called "aptamer" polynucleotide sequences which function as receptors for specific target biomolecule analytes from a wide range of biomolecule types including not only nucleotide sequences, but also polypeptide sequences, and which aptamers are obtainable by use of the well known SELEX (systematic evolution of ligands by exponential enrichment) in vitro selection technique in which very large numbers of random polynucleotide sequences are iteratively screened and amplified for binding to a particular target biomolecule analyte. In the case of a polypeptide macromolecular structure switch, suitable polypeptide analogue biomolecule binding aptamers are obtainable by use of well known vitro selection techniques such as Phage Display.

In yet another preferred form of the invention, there is provided a multi-macromolecular structure switch assembly of the invention in which a plurality of macromolecular structure switches of the invention is anchored to a substrate in substantially direct proximity to each other so that a plurality of switches can interact with each other, in one way or another. Thus for example the macromolecular structures may be mounted so that a competitor biomolecule may have different portions thereof bound to respective biomolecule analyte binding sites on two neighbouring macromolecular structures so as to lock them both in an open conformation. In such a case, displacement of the competitor biomolecule from the first macromolecular structure by a corresponding biomolecule analyte, would also result in unlocking of the second macromolecular structure against flipping to a closed conformation thereof. Such multi-macromolecular structure switch assemblies may be prepared by, for example, temporarily attaching them to a suitable template (for example, hybridizing to respective portions of a large macromolecular template) so as to provide an ordered assembly of the macromolecular structure switches, anchoring the macromolecular structure switches of the ordered assembly to the substrate, and then removing the template.

It will be appreciated that certain preferred forms of the macromolecular switches of the invention may be considered to be analogous, to a greater or lesser degree, to transistors in that they may provide a fundamental building block for logic circuits much as transistors do for silicon devices, and may conveniently be referred to as bio-transistors. Nevertheless it is not intended that the scope of the present invention should in any way be restricted to devices having specific transistor-like features or characteristics. For the avoidance of doubt, as used herein, the terminology "bio-transistor" is used to indicate transistor-type devices in which said macromolecule structure is substantially comprised of one or more biomolecules, whether or not such a bio-transistor device is used for interacting with other biomolecules, as well as devices in which the macromolecule structure is not a biomolecule at all, but is nevertheless capable of interacting, with a greater or lesser degree of specificity, with biomolecules. For the avoidance of doubt, the term "biomolecule" is used herein to encompass not only molecules found in living organisms, but any molecule having a character substantially similar to such molecules, howsoever, obtained. Thus, for example, biomolecule includes polypeptides whether made by processes having a biological character or purely organic chemistry synthetic processes, and polypeptides which are entirely artificial, i.e. are not found in nature. It should also be noted for the avoidance of doubt that references to "biomolecule" and "macromolecule" herein include both discrete individual molecules and more or less closely bound combinations of a plurality of discrete individual molecules. Thus, for example, an HJ (discussed in more detail elsewhere herein) is actually a combination of four nucleic acid strands (or like entities) bound together into a single macromolecule entity with four arms, each comprising parts of two strands bound to each other. It should also be noted that in relation to the devices of the present invention, as will appear from the description hereinbelow, some devices of the present invention are more transistor-like in nature and/or operation than others, and the term transistor is not intended to limit the scope of the present invention, but rather is used primarily as an aid to understanding.

It will be appreciated that the total output signal obtainable from a switch device of the present invention will depend on the number of the macromolecule structures used in each device. Preferably therefore the device of the present invention has a plurality of said macromolecule structures. Desirably there is used at least 5, more desirably at least 10, preferably at least 100, advantageously at least 1000, most conveniently at least 10,000, macromolecule structures, in each switch device.

It will be appreciated that said at least one electrochemical input device may be formed and arranged for applying such different ion binding conditions to said ion binding site of said macromolecular structure in any convenient manner which allows transmission of ion binding conditions. In general this involves retaining the macromolecular structure in proximity to said input device, in a "fluid-interfaceable condition". As used herein, the expression "fluid - interfaceable condition" means that the structure is supported in any convenient manner which would permit interaction between the macromolecular structure and a fluid medium. Conveniently the macromolecular structure may be anchored in a suitable manner to a substrate having a suitable form, so that said structure can interact with components present in a fluid medium with which the device is contacted in use of the device. Thus whilst the structure would generally be mounted on the surface of a solid substrate, it could also be embedded inside a more or less permeable substrate, which may moreover be more or less flexible, such as, for example a gel. Naturally in such a case the gel should be chosen as to have a pore size sufficiently large to enable the target components in the fluid medium to be able to penetrate the gel and come into contact with the structure. It is also important that the gel pore size should be sufficient to allow adequate freedom of movement of the structure between its first and second conformations. Furthermore the macromolecular structure could also in principle simply be suspended in solution within a cell or other vessel provided with said input device. Such a cell may moreover be defined by fluid impermeables walls, or in some cases, at least partly, by fluid permeable walls such as dialysis membrane. By suitable choice of the pore size of such a membrane it is moreover possible to entrap the macromolecular structure within a zone in direct contact with the electrochemical input device, with a sample solution containing target biomolecule analyte being contacted with the outside of the membrane.

It will of course also be appreciated that at least some macromolecular structures, especially biomolecular ones, may be susceptible to denaturation or other damage to a greater or lesser degree under certain conditions e.g. high and/or low pH, and it is therefore desirable that this should be taken into account in relation to the properties of any medium through which the macromolecular structure is supported and/or interfaced through with biomolecule analytes. Alternatively or additionally, macromolecular structures may be modified so as to stabilize them to a greater or lesser extent against denaturation or disintegration, for example by means of cross-linking of the hybridized polynucleotide sequences within one or more arms of an HJ, for example using a cross-linking agent such as psoralen. This can be useful where it is, for example, desired to remove biomolecules which have been bound to biomolecule analyte binding sites on an HJ, without loss of the HJ structure in order that it can continue to be used. More stable HJ structures are also obtainable by means of using synthetic polynucleotide analogues based on the use of size-expanded analogues of the natural bases (for example, using a three ring analogue of a bicyclic purine and a bicyclic analogue of a monocyclic pyrimidine as discussed by Liu et al in Science, Vol 302 Issue 5646, 868-871 (2003). (For the avoidance of doubt, unless the context specifically requires otherwise, the term HJ is intended to encompass modified HJs.)

In anchoring or tethering the biomolecules to an electrode surface, several factors should be borne in mind. The electrode can be chosen from any of an assortment of conductors or semiconductors. These range from metals such as silver, gold, platinum, titanium, tantalum, tungsten, aluminium, nickel, copper and alloys of these or other metals to semiconducting or conducting oxides, nitrides or mixed oxynitrides of metals such as titanium, nickel, iron, tin, indium, tantalum, strontium, iron, tungsten, niobium, iridium, molybdenum, hafnium, zinc, aluminium and zirconium and doped versions thereof. Electrodes may also be chosen from,materials such as carbon, conducting and semiconducting molecular organic systems, inorganic and organic charge transfer salts (e.g. tetrathiafulvalene-tetracyanoquinodimethane), and semiconducting materials such as gallium arsenide or conducting polymers such as polypyrrole, polythiophene or polyaniline.

In accordance with the present invention, a biomolecule may generally be anchored to an electrode or other suitable substrate using one or more of three principal approaches: Physisorption, chemisorption or covalent attachment.

Physisorption is procedurally relatively simple and relies on non specific interaction between areas of, for example, complementary charge or similar hydrophobicity. It tends not to impart controlled orientation and may be reversible under conditions of varying pH, ionic strength or solvent hydrophobicity.

Chemisorption is a more specific form of orientation since it relies on the favoured interaction between a surface and a particular moiety on a molecule, examples of such are the interaction between thiols and gold and the interaction between phosphonic acids and metal oxides.

Covalent attachment of biomolecules relies on the modification of an electrode surface so that it is intrinsically active to or can be activated to attach to specific, functional groups on the biomolecule. Suitable treatment might be the oxidation of carbon electrodes to form carboxylic groups on the surface which can be activated to bind with amine groups. Another example would be the use of a functional silane compound (for example an epoxide terminated silane) to form a self-assembled, covalently attached layer that can be used to covalently attach a polymer on, for example, a metal oxide. Specifically, aminosilane can be polymerised onto a metal oxide such as titanium dioxide to form a surface with active amino groups that can react with carbonyl functions on the surface of a biomolecule. In both such cases, the surface coating can be made sufficiently thin so as to not hinder electron transfer across the interface. Thus the amino group on the 5'-terminal of arm III of the HJ structure may itself be used for covalent bonding directly to a substrate. Alternatively a linking moiety such as a biotin molecule may be attached to the 5'-terminal of arm III of the HJ structure. Although it is generally more convenient to use the 5'-terminal of arm III of the HJ structure for anchoring, it is nevertheless also possible to effect anchoring via the 3'-terminal if desired.

Such techniques can moreover be combined to form a suitable surface for biomolecule anchoring. Thus, for example, a polyion such as poly-1-lysine can be physisorbed onto a charged electrode surface on the basis of charge pairing. As the poly-1-lysine is a polypeptide with amino side chains, the amino groups can be attached to activated carbonyls on a biomolecule or cross linked to a biomolecule in order to give covalent attachment. Chemisorption of a bifunctional alkyl thiol can convert a gold surface into a surface reactive to biomolecular binding. For example mercaptopropionic acid can be chemisorbed onto gold to give a self-assembled monolayer with carboxylic acid moieties reaching into the bulk solution. These carboxylic groups can be activated by carbodiimide chemistry to react with amine groups on a biomolecule.

Macromolecule structures can also be anchored by means of physical entrapment. This involves the formation of a polymer network or sol-gel or hydrogel substrate that has a pore size dimensioned so as to enable it to entrap a biomolecule during its formation. Although this can give a stable environment for the biomolecule, the surrounding matrix limits its interaction with other biomolecules to a greater or lesser degree. Also, since entrapment is not a covalent immobilisation it may be prone to leaching to a greater or lesser degree. Accordingly it is important to ensure that when a gel or other polymer network substrate is used, this has a suitable pore size and suitable cavity size for effectively retaining the structure within the substrate whilst allowing it sufficient freedom of movement between its first and second conformations, as well as allowing freedom of access of target biomolecules to the entrapped biomolecule structure. Suitable gels include polyacrylamide gels, polysaccharide hydrogels, for example carboxy-methyl dextran. It will be appreciated that when gels are used, the macromolecule structures can also be covalently bonded to the gel matrix in order to prevent escape thereof over a period of time. Furthermore, macromolecule structures can also be "anchored" by means of relatively strong non-covalent bonding e.g. biotin binding to streptavidin.

Various means may be used for reading of the conformational state of the macromolecular structure and/or the presence of a target biomolecule analyte bound to a said biomolecule analyte binding site. Thus it will be appreciated that where the target biomolecule analyte is labelled, then the bound analyte may simply be detected in the usual way by monitoring the presence of labelled material, after the removal of unbound material. Many different labelling systems including radio-labelling, fluorophore labelling (including quantum dots), redox labelling, and indirect labelling systems such as biotin incorporation with detection by streptavidin coated particles including nanoparticles, which could be used, are well known in the art.

In general, the different conformational states of the macromolecular structure of the macromolecular structures used in accordance with the present invention will correspond to different separation distances of particular (first and second) parts of the macromolecular structure, whereby changes in conformation state may be conveniently monitored by means of a separation sensitive signal transmission system. Such systems will generally include respective output device components mounted (directly or indirectly) on said first and second parts of the macromolecular structure. Preferably at least one said component is mounted directly on a said (first) part of the macromolecular structure. A (or the) second said output device component may be located on another part of the macromolecular structure (for example on another arm in the case of an HJ structure), or could instead be mounted on a target biomolecule analyte and thus will only be incorporated into the macromolecular structure when the biomolecule analyte becomes bound to said biomolecule analyte binding site.

The separation sensitive signal transmission system may in some ways be considered to be analogous to the data carrier signal portion of conventional transistor devices in that it has an input "connection"(s) to which is applied (an) input signal(s) received from one part of an associated circuit and an output "connection"(s) which passes an output signal to another part of a signal processing circuit, the relationship between these output and input signals being dependent on the electrochemical control signal input, whereby the data carrier signal portion provides an output signal containing data related to the control signal input(s). Thus said input and output signals received from and passed to respective parts of the signal processing circuit and passing through the separation sensitive signal transmission system, are used to carry data out of the "transistor".

Various forms of separation sensitive signal transmission system may be used in accordance with the present invention. Thus in one preferred form of the invention there is used a FRET system comprising a donor fluorophore mounted on the first part and an acceptor fluorophore mounted on the second part of the macromolecule structure. In this case an optical signal is "input" into the donor to excite the donor which then transfers energy (non-radiatively) to the acceptor which then provides an output optical signal. The energy transfer from the donor to the acceptor is sensitive to the separation therebetween, thereby affecting the intensity of the output signal. In order to allow the non-radiative energy transfer to take place, the donor and acceptor fluorophores must be in relatively close proximity - generally not more than 10 nm, advantageously from 0.2 to 10 nm, preferably in the range from 2 to 8 nm. When FRET does take place, as well as being indicated by the presence of an output signal from the acceptor fluorophore which is distinguishable from any donor fluorophore fluorescent radiation by having a longer wavelength, it can also be detected by a reduction in the donor fluorophore fluorescent radiation signal level. In another preferred form of the invention, there is used only a single fluorophore type in conjunction with a quenching agent. In this case an optical signal is input into the donor which then provides an output optical signal. The intensity of the output signal in this case is sensitive to the separation between the fluorophore and the quenching agent.

In accordance with the present invention, one embodiment of the input and output signals of the separation sensitive signal transmission system, is optical signals which can readily be transmitted through an aqueous medium in which the macromolecule structure is supported in use of the device, and which can be more or less readily integrated with suitable electronic, opto-electronic, or optical signal processing elements.

The data carrier signal inputs to the switch device of the invention may be produced at said one part of a signal processing circuit in any convenient manner depending on the form and nature of the signal processing circuit. In the case of a substantially electronic circuit, there may be used an electro-optical transducer, such as LEDs or lasers to produce optical radiation at a frequency suitable for use with the separation sensitive signal transmission system (as further discussed hereinbelow). This optical radiation may be delivered from the electro-optical transducer substantially directly into the solution in which the "input connection" component of the separation sensitive signal transmission system of the macromolecular structure is supported, or via a suitable waveguide.

The separation sensitive signal transmission system signal outputs from the switch device of the invention may correspondingly be received at said another part of the signal processing circuit using any suitable form of opto-electronic transducer which can collect the optical data carrier signal outputs and convert them into electrical signals for further processing.

Furthermore it is also possible to utilise non-separation sensitive systems for reading of the conformation state, including for example, systems based on physical change in the macromolecule structure, e.g. electrochemical impedance generally based on changes in capacitance and/or resistance and/or inductance of a macromolecule structure population. In addition there may also be used other physical parameter monitoring systems based on physical changes such as a change in thickness of a macromolecule structure population layer.

For the avoidance of doubt it should be understood that references to signal processing circuits include any kind and degree of signal conditioning, amplification and computational processing such as any one or more of inter alia logical combinations with corresponding data carrier signal outputs from other biological transistor devices, amplification, digital and analogue-type functions or other like electronic processing, conversion into graphical display outputs etc, etc.

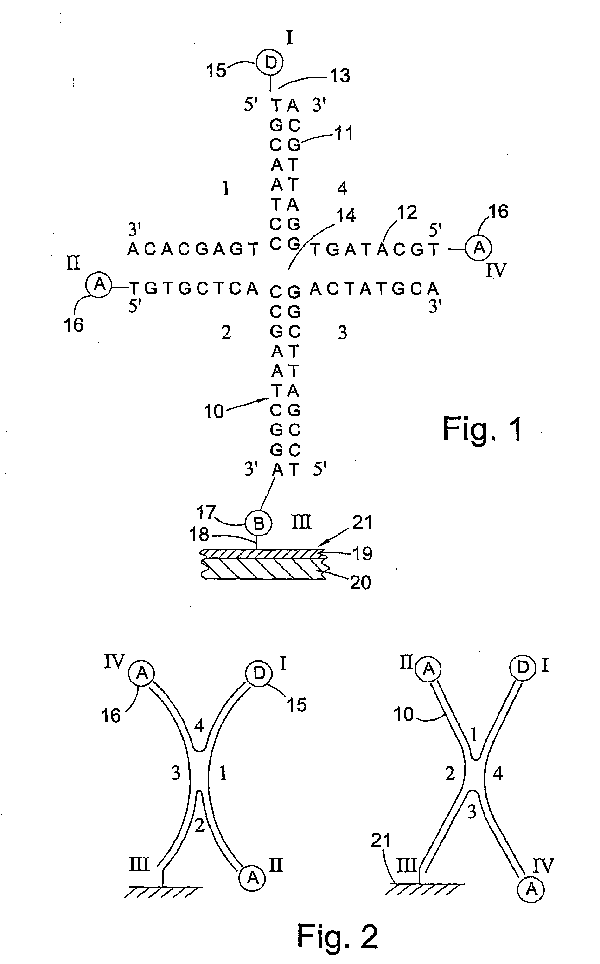

The transformation or flipping of the biological macromolecule structure between its different conformations is generally effected by applying suitable different ion binding conditions to the macromolecule structure ion binding site, depending on the general and/or particular nature of said macromolecule structure. In a preferred form of the invention the macromolecule structure comprises a Holliday-Junction structure (conveniently referred to herein as an HJ) having two pairs of arms extending out from a "branch point". In one, so-called "open" conformation the arms are fully extended or splayed out in a so-called "X-structure" which can be flipped to a "closed" or "stacked" conformation in which the two arms within each pair are substantially closer to each other than to the arms within the other pair of arms. In naturally occurring HJs there are generally two possible "closed" conformers with different combinations of arms within the pairs. It is also generally the case in naturally occurring HJs that the branch point migrates within the HJ structure. As further discussed hereinbelow, it is possible, by suitable choice of the polynucleotide sequences within the HJ branch point, to prevent substantially such migration. Similarly it is possible to favour one of the closed conformations to a greater or lesser extent for example, by using a branch point sequence combination such as that shown in Fig 1 of the accompanying drawings, the two closed conformations normally exist in a ratio of the order of 95:5. Whilst it would generally be simpler and more convenient to use HJs with just one substantially preferred closed conformation, it is nevertheless also possible to use HJs with two closed conformations of comparable population size. (It will be appreciated in such cases that the ion binding conditions for the two different closed conformers will be the same for each other, but of course different from those for the open conformer.) Thus the arms within said pairs are in closer proximity to each other in a "closed" one of the different conformations, than in any other combination of arms in any of the different conformations of the HJ structure - including another "closed" conformation where this is available.

Without wishing in any way to be restricted by the following, it is believed that the flipping of the HJ between open and closed conformations is affected by the ion binding conditions at an ion binding site in the region of the branch point, in at least two different ways including shielding of the negatively charged phosphate moieties of the sugar phosphate backbones within the branch point by positive ions so as to reduce repulsion therebetween, and specific binding of positive ions to the sugar phosphate backbones in the vicinity of the branch point. Furthermore these effects can be additive. In more detail we have found that various metal cations, including monovalent ones such as sodium and potassium, and divalent ones such as magnesium, which are small enough to enter the branch point can be used to effectively control switching, with divalent cations being effective at significantly lower concentrations than monovalent ones. We have also found that larger cations which are too large to enter the branch point, including those which are organic to a greater or lesser degree, including spermidine, are also effective in controlling switching. In this case it is believed they are effective through specific binding to the sugar phosphate backbone in the vicinity of the branch point. (On the other hand certain large cations e.g. tetramethylammonium do not appear to be effective in any way. Further suitable cations may, however, be readily determined by trial and error.) Furthermore we have found that using one type (e.g. spermidine) of ion at a level below that required for switching from an open to a closed conformation, can significantly reduce the minimum concentration level required of the other type (e.g. magnesium) of ion, in order to effect such switching.

Particularly convenient and useful are organometallic coordination complexes whose charge can be more or less readily varied by changes in redox potential, thereby facilitating control of ionic binding conditions given that we have there to be significant differences in ion concentration required for conformation flipping between ions of different charge. One such suitable ion system which may be mentioned is Fe(bipy)3²⁺ <- -> Fe(bipy)₃³⁺.

It will be appreciated from the above that the amount of cation(s) required will depend not only on the particular macromolecular structure used, and to some extent physical conditions (e.g. temperature), but also on the amount of macromolecular structure present. Suitable quantities in any given case may be readily determined by trial and error. Typically, though, for a macromolecular structure switch of the present invention containing 1 Micromolar of a suitable HJ, there would be required 100 micromolar of Mg²⁺ (Duckett et al EMBO Journal 9 (1990) 583-590).

It is also possible in some cases to configure macromolecular structure switches of the invention in a "normally open" or "normally closed" condition. Thus for example an HJ under high concentration ionic binding conditions corresponding to a closed conformation, but locked in an open conformation against flipping to a closed conformation, would automatically flip to its closed conformation as soon as the "lock" was released. It will be appreciated that by using in such a situation a locking element in the form of a competitor biomolecule bound to one or more biomolecule analyte binding sites, conformation flipping can be achieved directly by contacting the macromolecular structure switch with the target biomolecule analyte.

It will be appreciated that various different polynucleotide sequences may be used in the HJs in accordance with the present invention. Certain types of sequence are however preferred for various reasons including one or more of dimensional stability of the HJ structure, resistance to damage e.g. by free radicals, functioning of the data carrier signal transmission system, etc. Where a FRET system is used for data carrier signal transmission, it is preferred that that the donor and acceptor moieties are attached to a 5'-terminal T nucleotide in order to minimize local fluorophore-DNA quenching interactions. The length of the polynucleotide sequence between the branching point and the donor and acceptor fluorophores should moreover be sufficiently long to avoid steric hindrance to opening and closing of the HJ and to ensure that data carrier signal transmission is switched off in the open configuration of the HJ, without however being so long as to prevent data carrier signal transmission in the closed configuration. Where a FRET system is used, the donor and acceptor fluorophores should preferably be mounted so as to mount these at from 4 to 10 bases from the branch point, advantageously from 6 to 8 bases therefrom. It will also be appreciated that in the case of an HJ where substantial proportions of both closed conformers may be present upon switching from the open conformation, and it is desired to monitor flipping to each of the closed conformers, then an additional acceptor moiety may be provided on the other one of the HJ arms which pairs up with the Donor moiety bearing HJ arm in the second closed conformer, so that FRET monitoring may be carried out with each of the closed conformers. Moreover by using two Acceptor/Donor systems with different output wavelengths, it is possible to differentiate between switching to one or the other of the two closed conformers.

The anchored arm of the HJ is preferably of a length sufficient to provide at least one complete double-helix turn in order to provide a more stable support for the other arms. Dimensional stability may also be enhanced by using a so-called tripod mounting anchoring moiety which supports the anchored arm of the HJ in a substantially upright manner projecting out away from the substrate surface thereby reducing interference with the desired operation of the HJ structure in relation to one or more of interactions with target molecules, conformational switching etc (see for example Long B., Nikitin K., Fitzmaurice D., J. Am. Chem. Soc. 2003, 125, 5152-5160). Stability of the HJ structure may also be enhanced against separation of the polynucleotide strands of the arms, by binding them together with suitable bridging moieties. A relatively GC-rich area is advantageously provided in the HJ structure in order to protect critical parts of the HJ structure against damage from free radicals.

Having regard to the above there are preferably used polynucleotide sequences having a length of at least 13 bases, advantageously at least 21 bases, and generally not more than about 55 bases, although it will be appreciated that where HJs are used with polynucleotide sequence "extensions" for binding to target biomolecules, individual polynucleotide sequences may be somewhat longer. Where such extensions are used these should of course be of sufficient length to provide the required binding specificity, and typically will have a length of at least 13 bases, conveniently from 21 to 65 bases.

It should also be noted that HJ-like structures which have first and second, discretely different, conformations flippable in substantially similar manner to conventional HJs (consisting essentially of polynucleotide sequences), may also be constituted by molecular species in which a greater or lesser portion of the four HJ arm structures is comprised by non-polynucleotide material, and may be of any other organic and/or inorganic material which is not incompatible with the operation and application of the macromolecular structure, provided that the branch point of the HJ-like macromolecular structure has at least the four interacting polynucleoside dimer sequence elements of an HJ. (It will be appreciated that, whilst naturally occurring HJs are comprised by polynucleotides, an HJ-like macromolecular structure may also be comprised by the corresponding polynucleoside components of polynucleotides.) As discussed hereinbefore various different HJ macromolecular structures are known with differing ratios of the two possible closed conformers (see for example Hays et al Biochemistry 42 2003) 9586-97). One particularly suitable combination of polynucleotide dimer sequence elements which may be mentioned is: TC, GT, AG, and CA (as in the HJ shown in Figure 1). Accordingly any references to HJ structures herein are also intended to encompass such HJ-like structures unless the context specifically requires otherwise.

Another form of separation sensitive data carrier signal transmission system which may be used in accordance with the present invention comprises a fluorophore moiety providing input and output "connections" on said one part of the macromolecule structure and a quenching moiety provided on said second part of the macromolecule structure. In this case when the fluorophore receives energy from an exciting wavelength radiation source - corresponding to an input signal, it fluoresces sending out radiation at another wavelength - corresponding to an output signal which can be detected. The intensity of the output signal obtained is sensitive to any quenching agents which may be present in proximity to the fluorophore. Accordingly when the macromolecule structure is transformed from its first conformation to its second conformation, the quenching of the fluorescent radiation is changed thereby changing the output signal.

It will further be appreciated that quenching modulation can also be made use of-in conventional FRET systems (based on energy transfer from a donor fluorophore to an acceptor fluorophore) where energy transfer to the acceptor is quenched by close interactions between the donor and another material such as, for example, DNA. In this case hybridisation of a target molecule with part of the HJ can be used to reduce such interactions thereby reducing the quenching effect and increasing the ouput signal from the acceptor.

It should be noted that in order to maintain the integrity of the HJ structure i.e. prevent the polynucleotide sequences from separating from each other, suitable precautions should be taken. Thus, for example, the HJ structure may be maintained interfaced with an aqueous medium of HJ - compatible ionic strength. In general the medium should contain at least 20mM of salt. NaCl is a particularly convenient salt, but other salts such as KCl may also be used. Preferably the ionic strength is from 50 to 70mM. Nevertheless HJ integrity may also be maintained in other ways, for example, by means of cross-linking together of the ds polynucleotide strands (as further discussed hereinbelow) - even under less favourable ionic strength or other conditions.

With such a Holliday Junction structure, it is possible to change the ion-binding conditions so as to transform the structure between first and second confirmations in various ways including physical and/or chemical perturbations such as change of ionic strength and/or composition in the aqueous medium, changing a voltage applied to the aqueous medium (including changing from no applied voltage), changing the temperature or pressure of the aqueous medium, and changing the concentration of particular organic or inorganic molecules in the aqueous medium which have specific interactions (for example multiple hydrogen bonding)- as distinct from general physical interactions such as those due to ionic strength. Thus for example magnesium ions have a specific effect in switching individual HJ structures from an "open" configuration to a "closed" configuration, and are effective at significantly lower concentrations than monovalent cations such as Na⁺ and K⁺. In general a Mg²⁺ concentration of at least 100µM, conveniently from 10 to 50 mM is required to switch the HJ from open to closed configurations. Various other divalent and multi-valent cations may also be used in this way. Species such as Co(NH₃)₆]³⁺ which are redox active [Co(NH₃)₆]³⁺ + e- <=> [Co(NH₃)₆]²⁺ , may be used advantageously as they induce HJ switching at different concentrations, thereby enabling conformational switching electrically by means of changing said species from one redox state to another.

It is also possible to transform the biological macromolecular structure between said first and second conformations, by means of more or less specific interactions with positively charged biomolecules. Thus for example an HJ structure can also be transformed from its open to its closed conformation in the presence of polyamine species such as spermidine, and hexamminecobalt at as little as 100µm, conveniently around 2 to 5 mM.

In the case of other biological macromolecule structures involving one or more of macromolecule structures such as DNA, RNA, peptides, polypeptides, proteins and complexes formed by interactions between any of these, it may additionally be possible to change conformations by other means such as for example ionic strength and composition, solvent composition, light excitation, pH, molecular binding, redox switching and electric fields. Various specific examples of macromolecule structures which would be suitable are already known, see for example Rosengarth & Luecke (J Mol Biol 2003 Mar 7;326(5):1317-25)relating to the conformationally switchable polypeptide Annexin A1, and Backstrom et al (J Mol Biol 2002 Sep 13; 322(2):259-72) relating to the conformationally switchable polypeptide RUNX1 Runt. Various other suitable macromolecular structures may be more or less readily obtained by means of standard iterative screening and amplification such as SELEX (referred to hereinbefore) which is used with polynucleotide (including DNA and RNA) sequences, and Phage Display which is used for polypeptide sequences. (see for example Methods Enzymol. 364 (2003) 118-142) Suitable Polypeptide macromolecular structure switches according to the present invention also include inter alia metallo-enzymes that have discretely different conformations between which it is flippable upon binding to an ion. Particularly preferred macromolecular structures according to the present invention are those comprising at least one polynucleotide, and most preferably those comprising a Holliday Junction.

One well known form of macromolecule structure which may be mentioned here is the so-called "zinc finger" which comprises a polypeptide which has a conformationally transformable portion which switches from a generally straight extended configuration into a loop which can bind DNA (see for example Wolfe et al Biochemistry 42 (2003) 13401-9). In this case the first and second, portions of the separation sensitive data carrier signal transmission portion (e.g. donor and acceptor fluorophores of a FRET system) are mounted on first and second parts of the zinc finger structure at opposite end portions of said conformationally transformable portion so that they are moved towards and away from each other as the zinc finger switches conformation.

It will be appreciated that in the case of some biological macromolecule structures, said first and second conformations may correspond to more or less narrowly defined stable states, whereby said transformation therebetween operates in an essentially binary or digital manner thereby yielding binary or digital output signals (0 or 1 etc) from said signal transmission portion. Whilst it is a particular feature of the invention that the conformational flipping of the macromolecular structures can provide a substantially quantized output, it is also possible to use them to obtain analogue-form output signals. Thus, for example, analogue-type operation can be obtained in situations where the number of target molecules is significantly smaller than the number of macromolecule structures so that a positive output signal is obtained only from some of the macromolecule structures whereby the intensity of the output signal obtained depends on the concentration of the target molecule.

It will be appreciated that the macromolecular structure switches of the present invention may, in principle, be used simply in a manner essentially equivalent to that of conventional transistor devices, for example, as switches for logical processing or other purposes, with electrical control signal inputs to transform the macromolecule structure from one to the other of said first and second conformations etc. It is though a particular feature and advantage of the present invention that the switch devices provided herein have a bio-interactive capability. This may be realised in a number of different ways.

In one preferred form of the invention, the switch devices of the present invention, may be used in a mode in which the macromolecule structure conformation is transformed by a generally biological control signal input. The latter could be in the form of a biomolecule having a binding or complexing affinity which is more or less specific to at least part of said macromolecule structure, and which gives rise to conformation transformation (under at least some conditions e.g. particular ionic strength, electrical field, etc conditions), for example, as described hereinbefore with reference to a "normally open" macromolecular structure switch of the invention in which an HJ is held open by a "competitor" biomolecule (i.e. a biomolecule binding with an imperfect match) bound across two arms, and displaceable from at least one of them by means of competitive binding by a better matched target biomolecule analyte.

It should be noted that, in general, in at least some cases, depending on inter alia the nature of the macromolecule structure, transformation or flipping between different conformations may be obtainable by two or more different control signal inputs affecting ion binding conditions, for example, voltage and ionic strength, so that various combinations of different control signal inputs may be used which may have additive and/or subtractive effects (which may moreover be multi-dimensional), on the conformation transformations. Thus the effect of any given control signal input will also depend to a greater or lesser extent on any other (secondary) control signal inputs which may be present. This can also allow, for example, the sensitivity of the conformation device operation, to any desired control signal input, to be adjusted or tuned, by means of suitable choice of one or more other control signal inputs (and strength of biomolecular binding). In general the macromolecular structure switch is preferably configured so that the ionic binding conditions immediately before macromolecular structure switching, are close to the switching threshold for the ionic binding condition variable(s) being used to effect switching, in order to minimize the change in ionic binding conditions required for switching. In another preferred form of the invention discussed elsewhere herein, the macromolecular structure may be pre-biased towards a switched conformation, against the restraint of a conformation "locking" device.

It is also possible to combine control signal inputs to form logic gate elements such as AND, OR, NAND, XOR etc.

For example, an AND logic function would be:

| Control signal input | | Data carrier signal output |
|---|---|---|
| (1) | (2) | |
| - | - | - |
| - | + | - |
| + | - | - |
| + | + | + |

In another form of the invention, the bio-interactive capability is embodied in a somewhat different manner, as part of the data carrier signal transmission portion of the transistor device. In more detail, at least one of said first and second parts of said macromolecule structure is constituted by a discrete macromolecule part(s) which is connected to the rest of said macromolecule structure by complexing or affinity binding thereto. Thus in this form of the invention, it will be appreciated that the obtaining of any output signal under any control signal input conditions at all, is dependent upon the said discrete part(s) being connected to the rest of the macromolecule. Thus by using such a discrete macromolecule part which binds or complexes to the rest of the macromolecule with a greater or lesser degree of specificity, it is possible to detect the presence of a said discrete target macromolecule part in the solution in which the rest of the macromolecule is supported.

In a typical practical application, said discrete macromolecule part would be constituted by a target biomolecule which had been labelled with the respective ones of the input and output connections of the data carrier signal transmission portion (for example the donor or acceptor moieties of a FRET system), and the rest of the macromolecule structure would have a connector portion complexable or bindable with at least part of said target biomolecule with a greater or lesser degree of specificity. Thus in this case a functioning switch device of the invention would only be present, when the labelled target biomolecule had become bound or complexed to the rest of the macromolecule structure.

The presence of such a functioning switch device could then be selectively read out by means of varying a suitable control signal input to transform the macromolecule structure between conformations corresponding to disabled and enabled conditions of the separation sensitive signal transmission system of the macromolecular structure transistor device.

The present invention also provides a proto-biotransistor device comprising a biotransistor of the invention with only one of said first and second parts of the macromolecule structure mounting respective ones of the input and output connections of said data carrier signal transmission portion, and provided with a binding portion for selective binding, in use of the proto-biotransistor device, to a target biomolecule having the other of said first and second parts. When such a biomolecule ("labelled" with the other of said first and second parts of said macromolecule structure), binds to the proto-bio-transistor, it converts it into a functional biotransistor, whose operation can then be used to indicate the occurrence of such binding and thereby the presence of the target biomolecule.

The macromolecular structure switches of the invention can also be used in measuring the strength of particular binding interactions, by mounting respective partners of a binding interaction, on first and second portions of the macromolecule structure associated with respective ones of said first and second parts of the bio-transistor device, so that the binding together of said partners will inhibit, restrict or promote to a greater or lesser degree the normally obtainable transformation of the macromolecule between said first and second conformations thereof. By comparing the degree of transformation obtainable for a given control signal input and/or the level of control signal input required to achieve a given degree of transformation (and/or actual disruption of the binding), with that obtainable with suitable control bio-transistors (without any binding partners mounted thereon and/or with binding partners of known binding interaction strength), it is possible to obtain an indication of the strength of the binding in the target binding interaction. It is also possible to detect variations in binding strength which can arise when a polynucleotide designed for hybridising with a particular target DNA, hybridises with a variant of that DNA in which one or two bases are no longer matched properly resulting in a lesser degree of hybridisation and hence weaker binding. In such a case the variant may still provide a positive output signal, and the presence of a variant as distinct from the normal target DNA, may only be detectable by probing of the strength of the binding between the HJ extension and the DNA bound thereto. This is of particular significance where single nucleotide polymorphisms and the like are present.

In the case of an HJ bio-transistor the binding partners could in principle be mounted on any combination of the arms, but it will be appreciated that the mounting of the binding partners in certain positions on certain combinations of arms will have a stronger inhibitory effect on the conformation transformation, than others. In practice therefore the positioning of the binding partner mounting may be chosen in the light of the expected strength of the binding interaction and the strength etc of the control signal inputs available for challenging (reversing or overcoming) the target binding interaction.

It will be appreciated that, in the case of a macromolecule structure such as an HJ, the conformationally transforming macromolecule entity may be "extended" to a greater or lesser extent with molecular structure which is simply used, for example, to mount specific binding portions for binding with target biomolecules, for mounting binding partners of target binding interactions, for anchoring to the substrate, etc and which therefore need not necessarily be of the same nature as the conformationally transforming macromolecule entity, and, in at least some cases, need not be biomolecular in character.

Preferably there is provided a signal reading device such as for example a confocal scanning microscope or a photodiode with appropriate radiation wavelength sensitivity (see for example Physics of Semiconductor Devices, Sze S.M., John Wiley (1981)), coupled thereto for use in reading said signal outputs from said signal transmission device. A device element such as a photodetector e.g. a photodiode, is particularly convenient as this can be integrated into the substrate using integrated circuit microfabrication technology.

It will be appreciated that a variety of different kinds of signal transmission device may be used in the signal transmission bio-transistors of the invention. One particularly convenient form of signal transmission system is fluorescence resonance energy transfer (FRET) in which the input and output connections comprise donor and acceptor fluorophores. Suitable donor fluorophores which may be mentioned include Cyanine 3 (Cy3) and Fluorescein which are readily available commercially. Suitable acceptor fluorophores include Cyanine 5 (Cy5) and Rhodamine (Tetramethylrhodamine) which are readily available commercially. Cy3 has an exciting wavelength of 532 nm and Cy5 provides output radiation in the region of 580 to 600 nm. In general, with such signal transmission, a suitable light source is used to supply radiation at a first wavelength to the donor fluorophore which transfers out energy non-radiatively. If the acceptor fluorophore is within a predetermined range of the donor fluorophore (typically from 10 to 100 Angstrom), then the acceptor fluorophore will be able to convert energy received from the donor, into an output signal at a second wavelength which can be detected using suitable imaging spectroscopy apparatus.

Thus detection of an output signal at said second wavelength will indicate the presence of acceptor and donor fluorophores in the complex, in a conformation of said macromolecule structure in which the fluorophores are sufficiently close to each other for energy transfer therebetween. (Additional more detailed information on the separation between the donor and acceptor fluorophores, and/or for example the decay rate of fluorophores due to quenching thereof by other moieties (thereby enabling the use of single-fluorophore systems), can be obtained, if desired, by the use of more sophisticated output signal imaging analysis techniques such as frequency domain fluorescence lifetime imaging microscopy (FLIM)(Lacowicz, Principles of Fluorescence Spectroscopy, Princeton University Press) and Total Internal Reflection Fluorimetry (TIRF).

Various other forms of signal transmission device may also be used in accordance with the present invention including electrochemical, where for example the environment around the redox-active active site of an enzyme tethered to one of the arms of the biomolecule can be altered by the change in conformation of the biomolecule, designed to induce a relative movement of an enzyme inhibitory substance tethered to the other arm. This would affect the enzyme reactivity, and hence the catalytic current due to the enzyme reaction, which could produce an electrochemical signal output. Such a catalytic system would also provide signal output amplification.

In general there can be obtained a substantial change in the HJ conformation between the first and second conformations, resulting in a relatively large change in the separation of the signal transmission device component fluorophores (for example a change from a separation of the order of 1 nm to a separation of the order of 10 nm) so that two clearly distinct signal outputs can be reliably obtained, thereby providing a binary signal transmission capability, suitable for use in computational devices. It will be appreciated in this connection, that the sensitivity of any such separation sensitive readout system can be maximised if required, by careful choice of the positioning of the interacting components thereof on the relatively displaceable parts of the macromolecular structure.

As noted above, various different kinds of separation sensitive signal transmission system may be used in the bio-transistor devices of the invention as already discussed hereinbefore. The power supply connection and output signal connections used will naturally depend on the nature of the signal transmission. Thus, for example, where a FRET signal transmission is used, the connections would conveniently be in the form of optical waveguides optically coupled to the complex material. It will incidentally be appreciated that although, in principle, a component could utilise just a single complex, it would generally be more practicable to use more or less large numbers of complexes so as to provide signal levels which can be handled more easily and are less susceptible to individual error and thus more reliable.

The liquid medium mediated input signal may take various different forms. Thus for example in the case of a data carrier signal transmission system based on an enzyme or other biocatalyst in the HJ, then the input signal could be a mediator (e.g. NAD+/NADH, [Fe(CN)6]3-/4- or Fc/Fc+ where Fc is a substituted ferrocene such as ferrocene carboxylic acid. In this case signalling in/out may be effected via two electrodes separated spatially either side of the HJs, one electrochemically generating mediator and the other detecting or collecting mediator which had undergone redox reaction (both electrodes could be held at the same potential). One example of such a system uses a substituted Fc and GOD (glucose oxidase)as the enzyme. Given a large amount of glucose substrate, the rate of activity of the enzyme may be measured by the amount of Fc produced from electrogenerated Fc+, which is given by the current due to Fc ----> Fc+ at the collector electrode.

Although the devices of the present invention will normally be employed with said macromolecule structures supported in an aqueous medium, it may be more convenient for storage and/or shipping purposes to present the devices in a lyophilised form, and accordingly the present invention also encompasses such forms thereof.

In another aspect the present invention provides a switch as claimed in claim 1 comprising
a bio-interactive "transistor" device for controlling transmission of a data carrier signal, said device comprising
a) a biological macromolecule structure,
b) which structure has, at least, a first conformation and a second conformation different from said first conformation,
c) said structure being anchored to a fixed substrate in a fluid interfaceable condition,
d) so as to permit transformation between said first and second conformations in response to a control signal input,
e) first and second parts of said macromolecule structure mounting respective interacting, first and second portions of a separation sensitive data carrier signal transmission portion of said transistor device,
f) said first and second parts of said macromolecule structure having different separation distances in said first and second conformations of the structure, and
g) wherein different output signals are obtainable from said signal transmission portion in said first and second conformations of said structure, whereby different control signal inputs can provide different output signals.

Further preferred features and advantages of the invention will appear from the following detailed examples provided by way of illustration.

### Example 1 - Preparation of HJ "Transistor" Device

A homogeneous population of Holliday Junction (HJ) macromolecular structures is prepared using well-known techniques ("Nucleic Acids in Chemistry and Biology", 2nd Edition, Blackburn G.M. and Gait M.J.,). As shown in Fig. 1, the HJ structure 10 comprises four polynucleotides 11 with suitably chosen sequences such that respective end portions 12 bind together so as to produce an HJ structure 11 with four arms 13 radiating out from a branching point 14. In accordance with preferred identification terminology in the art, the four polynucleotides 11 are labelled 1, 2, 3 and 4, and the four arms 13 are labelled I, II, III and IV as shown in Fig. 1. (More particularly polynucleotide sequences 1 and 4 are known, sequence 2 is known save for the insertion of G-G adjacent the 3' end, and sequence 3 is known save for the insertion of C-C adjacent the 5' end.)

In more detail the polynucleotide 11 sequences are based on similar known sequences (Kallenbach N.R., Ma R.I., Seeman N.C. (1983) Nature 305 829-830), and are selected so as to provide an immobile junction which substantially prevents migration of the branching point 14. The polynucleotides 11 have additional moieties attached to respective ends thereof as follows. A Cyanine 3 (Cy3) fluorescent dye FRET donor moiety 15 (Amersham Biosciences UK Ltd, Little Chalfont, England) is attached to the 5¹ end of polynucleotide 1; a Cyanine 5 (Cy5) fluorescent dye FRET acceptor moiety 16 (Amersham Biosciences UK Ltd) is attached to the 5¹ end of each of polynucleotides 2 and 4, and a Biotin (bio-conjugated to Streptavidin), (or alternatively thiol conjugated to gold, or an amide conjugate linkage), anchoring moiety 17 is attached to the 3¹ end of polynucleotide 2. By providing in this way a FRET acceptor moiety 16 on each of the HJ structure arms II and IV, it will be appreciated that when the HJ structure 10 is switched (as further described hereinbelow) from its "open" configuration shown in Fig. 1 in which the arms 13 are all spaced apart, to either of the possible "closed" configuration conformers shown in Fig. 2, the FRET donor 15 will be brought into close proximity with a FRET acceptor 16. With the illustrated polynucleotide sequences it may be seen that the FRET donor and acceptor moieties 15, 16 are disposed at a separation from the branching point 14 such that in the "open" configuration, the FRET donor and acceptor moieties 15, 16 are spaced from each other by somewhat more that 100 Angstrom whereby no significant FRET energy transfer takes place therebetween, whilst in the "closed" configuration they are supported at around 50 to 80 Angstrom from each other, whereby FRET energy transfer from the donor to the acceptor takes place and radiation is emitted from the acceptor.

The bioconjugate anchoring moiety 17 is covalently bonded 18 using an amide linkage to a thin aminosilane coating 19 (generally 1 to 3 molecules thick) on a glass slide 20, thereby anchoring the HJ structure 10 to a substrate 21. (It will be appreciated that it would also be possible, to covalently bond the 5' end of the polynucleotide 2, or an intermediate part of the polynucleotide sequence 2 via the 2' position on the ribose moiety, directly to an aminosilane coating 19 using an amide linkage, or alternatively non-covalently anchor using biotin bound to strepavidin linked to an aminosilane coating.)

### Example 2 - Operation of HJ "Transistor" Device

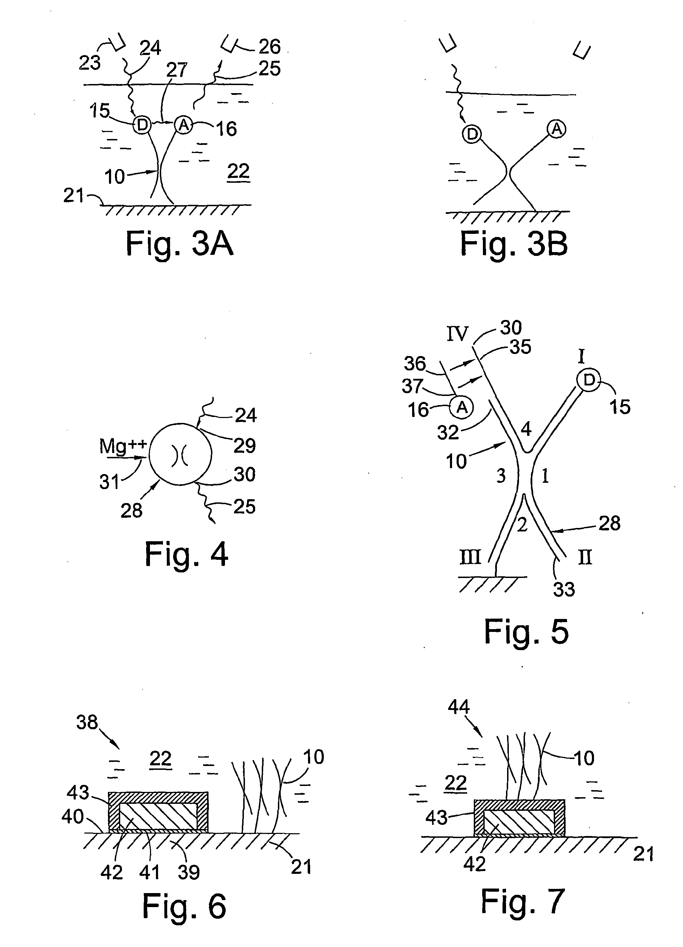

Fig. 3 shows schematically an HJ transistor device according to Example 1, comprising an HJ structure 10 anchored to a glass slide substrate 21. Typically the device comprises a homogeneous population of around 10,000 HJ structure molecular units supported in an aqueous buffer 22. The FRET donor moieties are excited by a laser source 23 providing 523 nm radiation 24 directed towards them and any output radiation 25 from the FRET acceptor moieties 16 is detected using a confocal scanning microscope (CSM) 26 (Ha T.J. et al Proc. Natl. Acad. Sci. USA 96 893-898 (1999)). When the aqueous buffer 22 contained magnesium ions (10 mM), the HJ structure 10 adopted a "closed" configuration as shown in Fig. 3A (corresponding to Fig. 2). In this configuration FRET energy transfer 27 to the FRET acceptor 16 takes place which results in an output radiation 25 which can be detected by the CSM 26. In this way a data carrier signal transmission 24, 27, 25 from the laser source 23 to the CSM 26 is enabled by a control signal comprising the magnesium ion concentration in the buffer 22.

When the magnesium ions are removed from the HJ structure 10 by washing with a suitable chelating agent such as EDTA, and the HJ structure 10 immersed in buffer 22 which is substantially free of magnesium ions, the HJ structure is switched to its "open" configuration as shown in Fig. 3B and no FRET energy transfer to the acceptor moiety 16 can take place whereby data carrier signal transmission is disabled. The signal processing nature of the above-described HJ device is represented diagrammatically in Fig. 4. The HJ device 28 (comprising an homogeneous HJ structure population anchored to a substrate and interfacing with a buffer in which it is supported) has a data carrier signal input 29, a data carrier signal output 30, corresponding to the laser radiation 24 used to excite the FRET donor moieties and the FRET acceptor moiety output radiation, and a control signal input 31 corresponding to the magnesium ion concentration provided in the buffer with which the HJ structure is interfaced.

### Example 3 - Use of HJ "Transistor" Device in Biomolecule Detection System

An HJ "Transistor" Device 28 is prepared with a homogeneous HJ structure 10 population generally similar to that in Fig. 1 except that in this case the 5¹ ends 32, 33 of polynucleotide 3 and 2 do not have FRET acceptors on them, and the 3¹ end 34 of polynucleotide 4 is extended beyond the 5¹ end 32 of polynucleotide 3 to provide a hybridising portion 35 for binding to a target polynucleotide 36 which has been labelled at its 3¹ end 37 with a FRET acceptor moiety 16. Thus in the absence of the target polynucleotide 36, it will be appreciated that there will be no FRET acceptor output radiation, irrespective of whether the HJ structure 10 is in its open or closed configuration. In the presence, though, of the target polynucleotide 36 there will, under hybridising conditions (Maniatis T., Fritsch E.F., and Sambrook J., (1982) Molecular Cloning: A Laboratory Manual, Cold Spring Harbor Press) be binding of the latter to hybridising portion 35 of the polynucleotide 4 resulting in attachment of the FRET acceptor 16 to the HJ structure 10 at a position on arm IV such that in the closed configuration shown in Fig. 5, FRET energy transfer from the FRET donor to the FRET acceptor can take place when the FRET donor is suitably excited. As in the previous example the HJ structure may be transformed from its "open" configuration to its "closed" configuration by application of a suitable Mg²⁺ ion concentration control signal input by interfacing the HJ structure with a buffer containing a Mg²⁺ ion concentration of 10 to 50 mM, whereupon transmission of a data carrier signal indicated by detection of a FRET acceptor output signal in response to input of a donor excitation signal, confirms presence of the target polynucleotide.

By using a homogeneous population with a large number of HJ structures, it will be appreciated that even if some of these assume the "wrong" conformer, others will adopt the "right" conformer in which the FRET donor and acceptor moieties are sufficiently close together for FRET energy transfer to take place (assuming both conformers are more or less equally available), whereby a detectable acceptor output signal level can still be obtained. Alternatively by suitable choice of the polynucleotide sequences, a more or less significant bias towards the conformer shown in Fig. 5, may be obtained, whereby FRET energy transfer can be obtained when the closed configuration is adopted and the FRET donor excited.

### Example 4 - Chemically Erasable HJ Memory Device Element

Fig. 6 shows a Memory Device Element 38 comprising a population of HJ structures 10 anchored to a silicon substrate 21 as before. Part 39 of the surface 40 of the silicon oxide substrate 21 is provided with a titanium based seed layer 41 in known manner comprising a titanium nitride layer having a thickness of around 0.1 µm on top of a titanium metal layer having a thickness of around 0.5 µm, (ULSI Technology, Sze S.M., McGraw-Hill (1996) and Silicon VLSI Technology Fundamentals, Practice and Modelling, Plummer J.D., Deal M.D., Griffin P.B., Prentice Hall (2000)). A platinum electrode 42 is attached to the titanium seed/adhesion layer 41 by means of sputtering thereonto, and is coated with an electrically conductive redox active polymer film 43 based on indol-5-carboxylic acid ( Mackintosh and Mount, J. Chem. Soc. Faraday Trans., 1994, 90, 1121-1125 and Mackintosh et al, J. Electroanal. Chem., 1994, 375, 163-168). (Alternatively we have found that a high quality Platinum electrode may be produced in a particularly convenient manner by electroplating onto a sputtered titanium nitride layer.) Prior to anchoring of the HJ structures 10 to the silicon oxide substrate 21, the conductive polymer film 43 is preloaded or "charged" with a population of Mg²⁺ ions by cycling the film between oxidised and reduced states thereof in the presence of Mg²⁺ ions. When the film 43 is oxidised H+ ions are expelled and when the film is reduced, Mg²⁺ ions are absorbed into the film 43 which thereby forms a reservoir of Mg²⁺ ions. (Mount and Robertson, Phys. Chem. Chem. Phys. (1999) 1 5169-77 )

Subsequently when a voltage differential of the order of 200mV is applied to the electrode 42 of the device 38, with a buffer 22 in the device 38 which is free from Mg²⁺ ions (or contains substantially less than 100µM, for example, not more than 50 µm of Mg²⁺ ions), so that the film 43 is changed from a reduced condition to an oxidised condition, Mg²⁺ ions are expelled from the film reservoir 43 into the buffer 22. This results in switching of the HJ structures 10 from their "open" position to their "closed" configuration, whereby data carrier signal transmission via the FRET donor and acceptor moieties is enabled as described hereinbefore. Thus the electrical voltage signal applied to the memory device element 38 acts as a memory write signal, whilst the FRET acceptor moiety output signal functions as a memory read signal. The memory device can subsequently be replaced by stripping the Mg²⁺ ions from the HJ structures by means of chelating agent such as, for example, EDTA.

### Example 5 - Chemically Erasable HJ Memory Device Element

Fig.7 shows another form of HJ Memory Device Element 44 in which the HJ structures 10 are anchored directly to the electrically conductive film coating 43 on a platinum electrode 42 similar to that in the Fig.6 embodiment. The HJ structures 10 are anchored by means of directly covalently bonding the amino group on the 5¹ terminal of the polynucleotide to a suitable functional group on the conductive film (most conveneniently a carboxylic acid group). In this case the conductive film coating 43 is "charged" with Mg²⁺ ions by means of redox state switching. The operation of this device 44 is essentially the same as that of the previous Example.

### Example 6 - Chemically Erasable HJ Memory Device Element

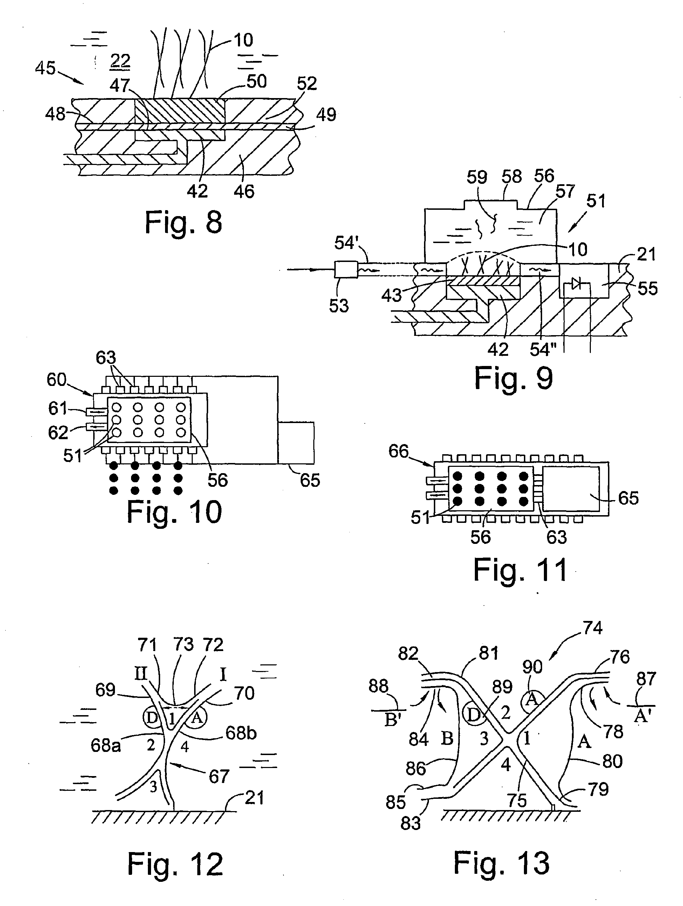

In this embodiment, illustrated in Fig. 8, a memory device element 45 has an aluminium metal electrode 42 embedded inside a silicon oxide substrate base layer 46. The upper surface 47 of the electrode 42 and surrounding substrate base layer surface 48 are then coated with a silicon nitride electrically insulating layer 49 using plasma enhanced chemical vapour deposition (PECVD) in known manner (ULSI Technology). A hydrogel permeation layer 50 is deposited on top of the conductive film 43. Suitable hydrogels or permeation layers are described in for example Electrophoresis 2000, 21, 157-164. These are typically 1-2 µm thick, made of agarose or poly(acrylamide) [Electrophoresis, 2002, 23, 1543-1550]. Typically an 2.5% aqueous solution of NuFix glyoxal agarose, is boiled, and then spincoated onto the conductive film, followed by curing at an elevated temperature (e.g. 37°C). Streptavidin or other chemical linking agents can also be incorporated into the hydrogel layer (e.g. by Schiff's base linkage between agarose glyoxal groups and a primary amine of the linking agent) to facilitate chemical linking of the DNA to the hydrogel. The permeation layer 50 enables electrophoretic transport of ions to the DNA.

An upper silicon oxide substrate layer 52 is deposited on the insulating layer 49 around the permeation layer 50 by means of PECVD in known manner (ULSI Technology). The hydrogel permeation layer 50 contains magnesium ionophores (a wide range is readily available e.g. from Sigma), and is charged with Mg²⁺ ions as generally described above. The HJ Structures 10 are anchored to the hydrogel layer 50 by means of for example biotin and streptavidin.

In this case application of an electrical voltage signal (typically several V, to the metal electrode 42 causes an electric field to be set up in the permeation layer 50 causing Mg²⁺ ions to be expelled therefrom into the buffer 22. In this case the buffer 22, as before should initially contain little or no Mg²⁺ ions, but should desirably have a high ionic strength in order to screen the HJ structures 10 from the induced electrostatic field, albeit once sufficient Mg²⁺ ions have been expelled from the permeation layer 50 into the buffer 22, the voltage signal to the electrode 42 may no longer be required and may be switched off, whereupon the Mg²⁺ ions released into the buffer may interact with the HJ structures 10 without interference from electric fields.

Where a high ionic strength buffer is used, this would conveniently have an ionic strength of the order of 10 to 20mM

### Example 7 - Biomolecule Detector Device

Fig. 9 shows an integrated biomolecule detector device 51 based on the HJ device element of Fig.7. In this case there is also incorporated into the semiconductor (with an oxide insulating layer) substrate 21 (by means of conventional semiconductor manufacturing techniques) a solid state laser light source element 52 coupled by a first optical waveguide 54' to the HJ structures 10 anchored to the redox active film 43 on the electrode 42. There is further incorporated a pn junction light detector 55 coupled by a second optical waveguide 54" to the HJ structures 10. A sample chamber 56 is mounted on the substrate 21 for holding a sample solution 57 in contact with the HJ structures 10, and has an inlet 58 for introduction of a sample solution 57 containing target biomolecules 59. In this case the HJ structures 10 are of the kind illustrated in Fig. 5, with polynucleotide extensions selectively hybridisable with portions of the target biomolecules 59.

In use of the detector device 51, a sample solution 57 is initially introduced into the sample chamber 56 under hybridising conditions (Maniatis et al).

After hybridisation has been completed, the sample solution is washed out and replaced by a substantially magnesium ion-free buffer 22 (<<100mM). A voltage is then applied to the electrode 42 in order to release Mg²⁺ ions from the redox active film 43, into the buffer 22. This results in the HJ structures being switched from their open configuration into their closed configuration, whereupon the laser source 52 is switched on and the output of the light detector 54 monitored. The presence of target biomolecules 59 in the sample solution, is indicated by the detection of an output signal from the light detector 54, the strength of the latter signal being generally proportional to the concentration of the target biomolecules in the sample (up to a level corresponding to saturation of the HJ structures and assuming a light detector 54 with a generally linear response, is used)

### Example 8 - Multielement Biomolecule Detector Devices

Fig. 10 shows schematically a multielement biomolecule detector device 60 provided with a single common sample chamber 56 for holding sample solution 57 in contact with the HJ structures of an array of biomolecule detector devices 51, each individual device 51 having a homogeneous HJ structure population with a different polynucleotide extension for hybridising with different target biomolecules. The chamber 56 has separate inlet and outlet parts 61, 62 for facilitating sample introduction, and replacement thereof with buffer etc. The device 60 is provided with electrical signal connections 63 for coupling 64 of the electrodes, laser source element and light detectors, to control circuitry 65. With this device it is possible to probe a sample for the presence of several different target biomolecules, simultaneously.

Fig. 11 shows a generally similar device 66 but with the control circuitry 65 integrated on-chip.

### Example 9 - Logic Gates

### A. Multiple - input OR gate

A biomolecule detector device according to Example 7 is used, in which instead of using a completely homogeneous HJ structure population, there is used a population with a number of different polynucleotide extensions. With this device the presence of any one or more of a number of corresponding different target biomolecules may be detected.

### B. AND gate (Type 1)

A biomolecule detector device based on the preceding Example (A) is used, in which the HJ structures with different polynucleotide extensions also have FRET acceptor moieties which have output radiation at different wavelengths, and there is used a light detector formed and arranged for detecting the simultaneous presence of two (or more) different FRET acceptor output radiation wavelengths.

### C. AND gate (Type 2)

A biomolecule detector device based on Fig. 10 or Fig. 11 is used in which the light detector output signals from the different devices 51 are electronically processed by the control circuitry 65 to detect the simultaneous presence of desired target biomolecule combinations.

### D. AND gate (Type 3)

A biomolecule detector device based on the proceeding Example A is used in which a first HJ structure population with a first polynucleotide extension for selectively hybridising with a first target biomolecule labelled with a first FRET donor moiety, has a first FRET acceptor moiety, and a second HJ structure population with a second polynucleotide extension for selectively hybridising with a second target biomolecule labelled with a second FRET donor moiety, has a second FRET acceptor moiety with an output radiation having a wavelength different from that of the first. The second FRET donor moiety has an excitation wavelength different from that of the first and corresponding to that of the output radiation of the first acceptor moiety so that an output signal from the device is only obtainable when both first and second target biomolecules have been bound to respective HJ structure populations.

### E. AND gate (Type 4)

A biomolecule detector device based on the proceeding Example A is used in which an HJ structure population has a first polynucleotide extension for selectively hybridising with a first target biomolecule labelled with a FRET donor moiety, and a second polynucleotide extension for selectively hybridising with a second target biomolecule labelled with a FRET acceptor moiety, so that an output signal from the device is only obtainable when both first and second target biomolecules have been bound to respective HJ structure populations.

### Example 10 - Biomolecule Interaction Probe Device

This is based on a device of the type shown in Fig. 9 with an HJ structure 67 as illustrated in Fig. 12. In this case polynucleotide sequences 2 68a and 4 68b, are provided with portions (biomolecule analyte binding sites) 69, 70 forming parts of arms I and II of the HJ structure 67, which bind to respective portions 71, 72 of a target biomolecule 73. With such a probe device, when the respective portions 71, 72 of the target biomolecule 73 are relatively close together, simultaneous binding of the target biomolecule 73 to both biomolecule analyte binding sites can only take place when the HJ structure 67 is in its closed conformation.

### Example 11 - Bioinformatic chip for SNP detection

This diagnostic device is based on a device of the type 60 shown in Fig. 10 with an array of 50 DNA "transistor" devices 51. A sample of patient tissue is fluorescently labelled (with acceptor dye) and then introduced to the device 60 via inlet 61 by microfluidics. The sample molecules would have equal opportunity to interact with all of the DNA "transistor" devices 51, each of which is designed with polynucleotide extensions 35 to bind specifically to a particular gene expression signature (RNA molecule). Where genes are expressed in the sample tissue, the corresponding RNA molecules will hybridise to the specific DNA "transistors" device 51 which can then be read by FRET as described above.

The 50 devices 51 are designed to detect a particular disease signature based on a known pattern of gene expression. This pattern may be quite complex and involve a combination of digital logic (is gene on or off?) and analogue measurements (degree of gene expression) to determine the characteristic signature. The necessary processing of the output signals from the individual devices 51, is carried out in a suitable processor provided in the control circuitry 65.

This chip offers physicians the ability to offer an on-the-spot diagnosis based on gene expression profiling, without lengthy delays and costly administration.

### Example 12 -NOR Gate

This is based on a device of the type shown in Fig. 9 with an HJ structure 74 as illustrated in Fig. 13. In this case polynucleotide sequence 1 75, is provided with biomolecule analyte binding sites 76,77 forming parts of arms I and II of the HJ structure 74, which can bind to respective portions 78, 79 of a first target biomolecule analyte A 80 which holds the HJ structure 74 in its open conformation and locks it against being flipped into its closed condition. Similarly polynucleotide sequence 3 81, is provided with biomolecule analyte binding sites 82,83 forming parts of arms III and IV of the HJ structure 74, which bind to respective portions 84, 85 of a second target biomolecule analyte B 86 which also holds the HJ structure 74 in its open conformation. Thus if either or both of the first and second target biomolecule analytes A 80 and B 86 is present in the sample, the HJ structure will be locked against flipping, and flipping from the Open conformation to the closed conformation will only be possible, when neither target biomolecule analyte is present in the sample. As before the conformational state of the HJ structure 74 is conveniently read out using a FRET system with Donor and Acceptor fluorophores 89,90 which can only provide a FRET output signal when the HJ structure 74 is in its closed conformation.

### Example 13 -AND Gate

This is based on a device of the type shown in Fig. 13, in which the target biomolecule analytes A 80 and B 86 constitute competitor biomolecules pre-bound to the HJ structure. In this case when a first target biomolecule analyte A' 87 binds to one 76 of the biomolecule analyte binding sites 76,77 displacing competitor biomolecule A 80 therefrom, it partially enables flipping of the HJ structure 74 to its closed conformation. Such flipping cannot however take place until a second target biomolecule analyte B' 88 also binds to one 82 of the biomolecule analyte binding sites 82,83 displacing competitor biomolecule B' 86 therefrom. Thus the FRET system comprising Donor and Acceptor fluorophores 89,90 which can only provide a FRET output signal when the HJ structure 74 is in its closed conformation, will provide negative ouput signals when only target biomolecule analyte A' 87 or only target biomolecule analyte B' 88, is present, or when neither is present, and will only provide a positive output signal when both target biomolecule analytes A' 87 and B' 88, are present.

### Example 14 - Redox Chip Switching of HJ Macromolecular Structure Switch in Solution

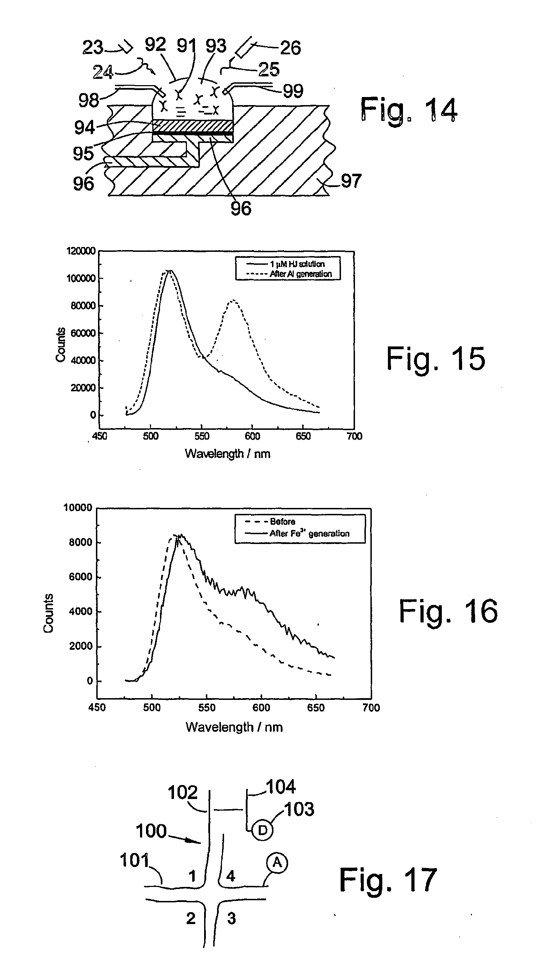

Switching of the macromolecular structure switches was studied using a population of HJ macromolecular structures supported in a droplet of solution placed on a substrate with an electrode as illustrated schematically in Fig. 14. In more detail Fig. 14 shows a population of HJ structures 91 provided with a FRET system (donor and acceptor moieties as described in the preceding Examples) in a 40 µL droplet 92 of solution (1 µM HJ concentration) in an aqueous TRIS buffer 93 (20 mM TRIS buffer at pH 7.5 containing 50 mM NaCl) supported on a platinum electrode 94 electroplated onto a titanium nitride adhesion layer 95 provided on an aluminium electrode 96 embedded using a damascene process in a silicon oxide base layer 97. A Platinum/Iridium counter electrode 98 and a silver/silver chloride reference electrode 99 are also contacted with the solution 92. The switching of the HJs was monitored using a laser source 23 and confocal scanning microscope 26 as described with reference to Example 2 hereinbefore.

### A. Switching using Iron Bypyridine Chloride

0.25 mM Fe (bipy)₃Cl₂ was added to the HJ solution and FRET spectroscopy carried out. The dashed curve in Fig. 15 shows the spectrum obtained (no FRET taking place). A potential sequence of +1.0 V vs Ag/AgCl (Ag/AgCl is -45 mV vs SCE) for 3 minutes and then +0.5 V vs Ag/AgCl for 3 minutes was then applied in order to convert the Fe from Fe²⁺ to Fe³⁺, following which FRET spectroscopy was again carried out and the solid curve in Fig. 15 shows the spectrum obtained. In this case FRET can be seen to be taking place confirming that switching of the HJs from the open to the closed conformation had taken place.

### B. Switching using Aluminium ions

Using a similar arrangement to that shown in Fig. 14 but with an Aluminium electrode (i.e. without the Platinum electrode 94 on a TiN₃ layer 95), switching using Al³⁺ ions was studied. FRET spectroscopy was initially carried out on the HJ solution and the solid curve in Fig. 16 shows the spectrum obtained (no FRET taking place). A potential ramp (2mV/s) was applied to the aluminium working electrodes from +0.3 V vs Ag/AgCl (Ag/AgCl is -45 mV vs SCE) up to +0.9 V generating Al³⁺ ions and releasing them into the HJ solution, following which FRET spectroscopy was again carried out and the dashed curve in Fig. 16 shows the spectrum obtained. In this case the difference in the before and after spectra shows that the Al³⁺ ions generated are interacting with the HJs.

### Example 15 - Operation of Biomolecule Detection System

To demonstrate that the HJ can be used as a biomolecular detector and switch, we prepared a 4 stranded HJ 100 illustrated schematically in Figure 17. The HJ 100 was substantially similar to that in Example 1 and Figure 1 but with strand 1 replaced by an extended 56mer polynucleotide sequence ss1 (5' tggatggtgtaaagttgaaaaaagataaactgaacccacatgcaatcctgagcaca 3') (and without any Donor fluorophore thereon) 101 (and with an Acceptor fluorophore 16 only on the polynucleotide 4 strand), so as to provide a ss biomolecule analyte binding site 102 on arm I. The fluoresceine Donor fluorophore-labelled 103 (at the 5' end of ss5) target biomolecule analyte 40mer ss5 (5' tgtgggttcagtttatcttttttcaactttacaccatcca 3'; Mus musculus neutropilin (Nrp)) 104, was contacted with the 4 stranded HJ 100 (in its closed conformation), under hybridizing conditions in a 20 mM TRIS buffer at pH 7.5 containing 50 mM NaCl and 5 mM MgCl₂ at 75 °C for 1 hour.

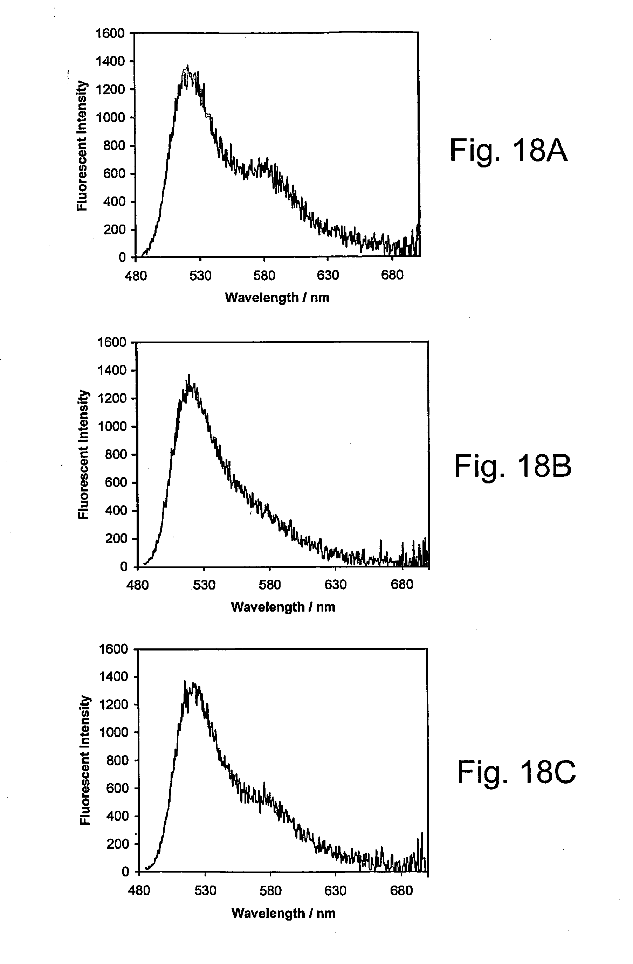

The resulting hybridized macromolecular structure was then subjected to two switching cycles as follows: closed-open, and open-closed, by first removing the Mg²⁺ ions by means of passing the solution through a spin column, and subsequently adding MgCl₂ back in again to restore the original concentration thereof. Fig. 18A shows the fluorescence spectrum obtained initially, and Figs 18B and 18C after each of the two switch cycles, respectively. As may be seen The FRET output signal obtained after the second cycle was substantially unchanged from that before the first cycle, with no FRET output signal being obtained after the first cycle, thereby confirming the reversibility of the switching operation and the stability of the hybridization of the biomolecule analyte 104 to the biomolecule analyte binding site 102 of the HJ 100.

### Example 16 - Stability of HJ Macromolecular Structure Switch

The stability of the macromolecular structure switches over time and with temperature changes was studied by means of fluorescence measurements carried out over a period of time on a population of HJ macromolecular structures in solution (40 µL of 1 µM HJ in a 20 mM TRIS buffer at pH 7.5 containing 50 mM NaCl and 5 mM MgCl₂). In more detail the solution was maintained at a base temperature of 25°C, with gradual heating up to 40°C on the first day and natural cooling back down again, and similar successive heating cycles up to 40°C, 45°C, and 80°C on day 2. Fluorescence measurements were obtained immediately before and after each of the first three cycles, and then on day 3 and day 24. In each case a substantially similar Acceptor fluorescence peak characteristic of the Donor to Acceptor FRET of the closed conformer, was obtained.

### Example 17 - Reversible Switching of HJ Macromolecular Structure Switch in Solution

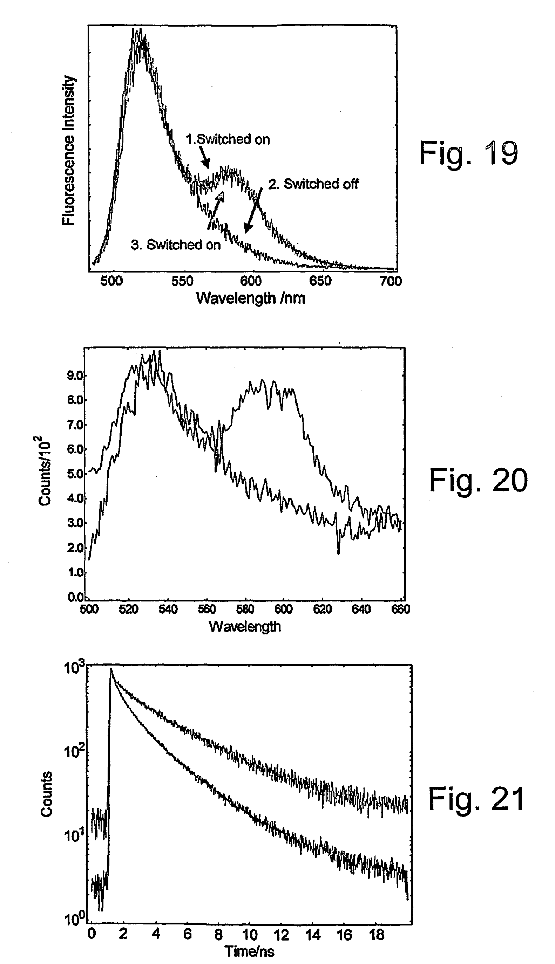

The reversible switching of the macromolecular structure switches was studied by means of fluorescence measurements carried out on a population of HJ macromolecular structures in solution prepared as before (40 µL of 1 µM HJ in a 20 mM TRIS buffer at pH 7.5 containing 50 mM NaCl and 5 mM MgCl₂). The macromolecular structure switches were subjected to two switching cycles as follows closed-open, and open-closed, by first removing the Mg²⁺ ions by means of passing the solution through a spin column, and subsequently adding MgCl₂ back in again to restore the original concentration thereof. Fig. 19 shows fluorescence data spectra obtained initially, and then after each of the switch cycles. As may be seen The FRET output signal obtained after the second cycle was substantially unchanged from that before the first cycle, with no FRET output signal being obtained after the first cycle, thereby confirming the reversibility of the switching operation.

### Example 18 - Switching of HJ Macromolecular Structure Switch Anchored to Substrate

A population of HJs 10 labelled with Donor and Acceptor fluorophores 15,16 and generally similar to that illustrated in Fig.3A, in a Tris buffer (20 mM Tris at pH 7.5) containing Magnesium ions (5mM MgCl₂), was attached covalently via a carboxylic linkage to a glass slide coated with carboxymethyl dextran (CMD). In more detail, Pre-silanised microscope glass slides were immersed in a mixture of dextran (10 mg/ml) and NaOH (100 mM) for 24 hours followed by rinsing in distilled water and drying in a centrifuge. The dextran coated slides were next submerged in a mixture of bromoacetic acid (1 M) and NaOH (2 M) for another 24 hours and again followed by another washing and drying step. To activate the dextran slides, the slides were soaked in a mixture of freshly prepared NHS (n-hydroxysuccinimide, 20 mM) and EDC (1-(3-dimethylaminopropyl)-3-ethyl-carbodimide), 200 mM) in PBS for 30 minutes. The slides were subsequently washed in deionised water and dried. Once activated, the slides were ready for spotting with HJs. Deactivation of excess reactive carboxyl groups was performed by rinsing the resulting slide with HJs attached thereto, with ethanolamine (10 mM).

The Tris buffer solution was then passed through a spin column so as to remove the Magnesium (Mg²⁺) ions, and returned to the HJs anchored to the slide. Fluorescence measurements were carried out before and after removal of the Magnesium ions from the buffer, using Time Correlated Single Photon Counting (TCSPC), using a pulsed laser Titanium Sapphire femtosecond laser system.
Fig. 20 shows a spectrum of intensity (measured in Counts/10²) against wavelength. The upper curve was obtained before removal of Mg²⁺ and the lower curve after. The former clearly shows a radiation peak corresponding to Acceptor fluorescence, indicating that FRET has taken place from the Donor to the Acceptor and that the HJs are in their closed conformation. The Acceptor fluorescence peak is absent from the lower curve indicating that FRET is no longer taking place from the Donor to the Acceptor and that the HJs are in their open conformation in the absence of Mg²⁺.
Fig. 21 shows the decay of the Donor fluorescence at a single wavelength (539nm) following excitation thereof by a single laser pulse. The lower curve corresponds to the presence of Mg²⁺ and the upper curve to the absence thereof, showing a much faster decay of the Donor fluorescence due to FRET from the Donor to the Acceptor for the closed conformer which obtains when Mg²⁺ is present, than for the open conformer which obtains when Mg²⁺ is absent and with which FRET cannot take place. Thus the measurements shown in Fig. 21 reinforce those shown in Fig. 20 and other similar measurements referred to hereinbefore.

## Claims

1. A macromolecular switch suitable for use in a data acquisition and/or processing device, comprising: a Macromolecular structure having at least one ion binding site, and separately at least one biomolecule analyte binding site formed and arranged for binding to a predetermined target biomolecule analyte, said macromolecular structure being flippable between a plurality of discretely different conformations corresponding to different ion binding conditions at said at least one ion binding site, at least in the absence of any other binding to said macromolecular structure, and further said macromolecular structure being provided with at least one electrochemical input device formed and arranged for applying such different ion binding conditions to said ion binding site in response to corresponding external input signals.

2. A switch as claimed in claim 1 wherein said at least one input device is formed and arranged so as to be operable under the control of electrical external input signals.

3. A switch as claimed in claim 1 or claim 2 wherein said electrochemical input device comprises an ion reservoir and an ion reservoir control device formed and arranged for at least one of ion capture into and ion release from, said ion reservoir.

4. A switch as claimed in claim 3 wherein said ion reservoir control device comprises a redox system formed and arranged for said ion capture into and ion release from, said ion reservoir.

5. A switch as claimed in any one of claims 1 to 4 wherein said at least one electrochemical input device is formed and arranged for reversible switching between said different ion binding conditions,

6. A switch as claimed in claim 5 wherein are provided at least two said biomolecule analyte binding sites.

7. A switch as claimed in any preceding claim wherein at least one said biomolecule analyte binding site has a competitor biomolecule, bound thereto, which competitor biomolecule is substitutable by a said target biomolecule analyte in use of the switch, by means of competitive binding.

8. A switch as claimed in claim 7 wherein said competitor biomolecule is bound to said macromolecular structure so as to lock said structure in one of said conformations against flipping to a different said conformation,

9. A switch as claimed in any one of claims 1 to 8 wherein said macromolecular switch is provided with at least one output device component of an output signal transmission system formed and arranged for use in reading of at least one of: the conformational state of the macromolecular structure and the presence of a target biomolecule analyte bound to a said biomolecule analyte binding site.

10. A switch as claimed in claim 9 wherein said component comprises part of a separation sensitive output device.

11. A switch as claimed in claim 9 or claim 10 wherein said output device component comprises a fluorescent label.

12. A switch as claimed in claim 11 wherein said fluorescent label is suitable for use in a separation sensitive signal transmission system.

13. A switch as claimed in claim 12 wherein said separation sensitive signal transmission system is a fluorescence resonance energy transfer (FRET) system.

14. A switch as claimed in any one of claims 9 to 13 wherein said macromolecular structure is coupled to at least one solid state data signal processing component.

15. A switch as claimed in claim 14 wherein said at least one solid state data signal processing component comprises a component for transforming an optical signal into an electronic signal,

16. A switch as claimed in any one of claims 1 to 15 wherein said macromolecular structure is coupled to at least one solid state data processing circuit, for transferring data substantially directly thereto.

17. A switch as claimed in any one of claims 1 to 16 wherein said macromolecular structure is captively retained in proximity to said at least one input device.

18. A switch as claimed in claim 17 wherein said macromolecular structure is anchored to a solid substrate.

19. A switch as claimed in any one of claims I to 18 wherein said macromolecular structure comprises an Holliday junction (HJ)-like structure in which at least part of the four HJ arm structures is comprised by non-polynucleotide material, which is not incompatible with the operation and application of the conformation switching of the macromolecular structure, and wherein the branch point of the HJ-like macromolecular structure has at least the four interacting polynucleoside dimer sequence elements of an HJ) branch point.

20. A switch as claimed in claim 19 wherein said four interacting polynucleoside dimer sequence elements of an HJ branch point are comprised by: TC, GT, AG, and CA.

21. A switch as claimed in any one of claims 1 to 18 wherein said macromolecular structure comprises at least one polynucleotide.

22. A switch as claimed in claim 21 wherein said macromolecular structure is a Holliday-Junction.

23. A switch as claimed in any one of claims 1 to 18 wherein said macromolecular structure comprises at least one polypeptide.

24. A switch assembly comprising a plurality of switches according to any one of claims 1 to 23, provided with an input signal device formed and arranged for applying external input signals to said input devices of said switches, in temporally separated manner.

25. A switch assembly comprising a plurality of switches according to any one of claims 1 to 23, wherein said switches have different biomolecule analyte binding sites and are disposed in substantially direct proximity to each other so that one said switch can interact with a neighbouring said switch.

26. A switch assembly as claimed in claim 25 wherein said switches are formed and arranged so that said one switch is coupled to a said neighbouring switch via a biomolecule binding to respective biomolecule analyte binding sites thereof.

27. A switch as claimed in claim 1 comprising a bio-interactive "transistor" for controlling transmission of a data carrier signal, said device comprising
a) a biological macromolecule structure,
b) which structure has, at least, a first conformation and a second conformation different from said first conformation,
c) said structure being anchored to a fixed substrate in a fluid interfaceable condition,
d) so as to permit transformation between said first and second conformations in response to a control signal input,
e) first and second parts of said macromolecule structure mounting respective interacting, first and second portions of a separation sensitive data carrier signal transmission portion of said transistor device,
f) said first and second parts of said macromolecule structure having different separation distances in said first and second conformations of the structure, and
g) wherein different output signals are obtainable from said signal transmission portion in said first and second conformations of said structure, whereby different control signal inputs can provide different output signals.

## Patentansprüche

1. Makromolekularer Schalter, der für eine Verwendung in einer Datenerfassungs- und/oder verarbeitungsvorrichtung geeignet ist, wobei er Folgendes umfasst: eine makromolekulare Struktur, die wenigstens eine Ionenbindungsstelle und gesondert wenigstens eine Biomolekülanalyt-Bindungsstelle hat, geformt und angeordnet zum Binden an einen vorbestimmten Ziel-Biomolekülanalyten, wobei die makromolekulare Struktur zwischen mehreren diskret unterschiedlichen Konformationen umgeschaltet werden kann, die unterschiedlichen Ionenbindungszuständen an der wenigstens einen Ionenbindungsstelle entsprechen, wenigstens in der Abwesenheit jeglicher anderer Bindung an der makromolekularen Struktur, und wobei ferner die makromolekulare Struktur mit wenigstens einer elektrochemischen Eingabevorrichtung versehen ist, die dafür geformt und angeordnet ist, als Reaktion auf entsprechende äußere Eingabesignale solche unterschiedlichen Ionenbindungszustände an die Ionenbindungsstelle anzulegen.

2. Schalter nach Anspruch 1, wobei die wenigstens eine Eingabevorrichtung so geformt und angeordnet ist, dass sie unter der Steuerung von elektrischen äußeren Eingabesignalen bedient werden kann.

3. Schalter nach Anspruch 1 oder Anspruch 2, wobei die elektrochemische Eingabevorrichtung ein Ionenreservoir und eine Ionenreservoir-Steuerungsvorrichtung umfasst, geformt und eingerichtet für wenigstens einen der Vorgänge Ioneneinfangen in das Ionenreservoir und Ionenfreisetzen aus demselben.

4. Schalter nach Anspruch 3, wobei die Ionenreservoir-Steuerungsvorrichtung ein Redoxsystem umfasst, geformt und eingerichtet für das Ioneneinfangen in das Ionenreservoir und das Ionenfreisetzen aus demselben.

5. Schalter nach einem der Ansprüche 1 bis 4, wobei die wenigstens eine elektrochemische Eingabevorrichtung für ein umkehrbares Schalten zwischen den unterschiedlichen Ionenbindungszuständen geformt und angeordnet ist.

6. Schalter nach Anspruch 5, wobei wenigstens zwei Biomolekülanalyt-Bindungsstellen bereitgestellt werden.

7. Schalter nach einem der vorhergehenden Anspruche, wobei wenigstens eine Biomolekülanalyt-Bindungsstelle ein an dieselbe gebundenes Konkurrenz-Biomolekül hat, wobei das Konkurrenz-Biomolekül bei Anwendung des Schalters mittels einer konkurrierenden Bindung durch einen Ziel-Biomolekülanalyten ersetzt werden kann.

8. Schalter nach Anspruch 7, wobei das Konkurrenz-Biomolekül an die makromolekulare Struktur gebunden ist, um so die Struktur in einer der Konformationen gegen ein Umschalten zu einer anderen Konformation zu sichern.

9. Schalter nach einem der Ansprüche 1 bis 8, wobei der makromolekulare Schalter mit wenigstens einem Ausgabevorrichtungsbaustein eines Ausgabesignalübertragungssystems versehen ist, geformt und angeordnet für eine Verwendung beim Auslesen wenigstens eines der folgenden Zustände: des Konformationszustandes der makromolekularen Struktur und des Vorhandenseins eines Ziel-Biomolekülanalyten, der an die Biomolekülanalyt-Bindungsstelle gebunden ist.

10. Schalter nach Anspruch 9, wobei der Baustein einen Teil einer trennungsempfindlichen Ausgabevorrichtung umfasst.

11. Schalter nach Anspruch 9 oder Anspruch 10, wobei der Ausgabevorrichtungsbaustein eine Fluorenszenzmarkierung umfasst.

12. Schalter nach Anspruch 11, wobei die Fluorenszenzmarkierung für eine Verwendung in einem trennungsempfindlichen Signalübertragungssystem geeignet ist.

13. Schalter nach Anspruch 12, wobei das trennungsempfindliche Signalübertragungssystem ein Fluoreszenresonanz-Energietransfer-(FRET-) System ist.

14. Schalter nach einem der Ansprüche 9 bis 13, wobei die makromolekulare Struktur an wenigstens einen Festkörper-Datensignalverarbeitungsbaustein gekoppelt ist.

15. Schalter nach Anspruch 14. wobei der wenigstens eine Festkörper-Datensignalverarbeitungsbaustein einen Baustein zum Umformen eines optischen Signals in ein elektronisches Signal umfasst.

16. Schalter nach einem der Ansprüche 1 bis 15, wobei die makromolekulare Struktur an wenigstens einen Festkötper-Datensignalverarbeitungsschaltkreis gekoppelt ist, um Daten im Wesentlichen unmittelbar an denselben weiterzuleiten.

17. Schalter nach einem der Ansprüche 1 bis 16, wobei die makromolekulare Struktur unverlierbar in der Nähe der wenigstens einen Eingabevorrichtung festgehalten wird.

18. Schalter nach Anspruch 17. wobei die makromolekulare Struktur an einem massiven Substrat verankert ist.

19. Schalter nach einem der Ansprüche 1 bis 18, wobei die makromolekulare Struktur eine Struktur in der Art einer Holliday-Struktur (Holliday junction - HJ) umfasst, wobei wenigstens ein Teil der vier HJ-Armstrukturen aus Nicht-Polynucleotid-Material besteht, das nicht unverträglich ist mit dem Betätigen und Anwenden des Konformationsschaltens der makromolekularen Struktur, und wobei der Verzweigungspunkt der HJ-artigen makromolekularen Struktur wenigstens die vier wechselwirkenden Polynucleosid,Dimer, Sequenzelemente eines HJ-Verzweigungspunktes hat

20. Schalter nach Anspruch 19, wobei die vier wechselwirkenden Polynucleosid-Dimer-Sequenzelemente eines HJ-Verzweigungspunktes aus TC, GT, AG und CA bestehen.

21. Schalter nach einem der Anspruche 1 bis 18, wobei die makromolekulare Struktur wenigstens ein Polynucleotid umfasst.

22. Schalter nach Anspruch 21, wobei die makromolekulare Struktur eine Holliday-Struktur ist.

23. Schalter nach einem der Ansprüche 1 bis 18, wobei die makromolekulare Struktur wenigstens ein Polypeptid umfasst.

24. Schalterbaugruppe, die mehrere Schalter nach einem der Ansprüche 1 bis 23 umfasst, versehen mit einer Eingabesignalvorrichtung, dafür geformt und angeordnet, äußere Eingabesignale, auf eine zeitlich getrennte Weise, an die Eingabevorrichtungen der Schalter anzulegen.

25. Schalterbaugruppe, die mehrere Schalter nach einem der Ansprüche 1 bis 23 umfasst, wobei die Schalter unterchiedliche Biomolekülanalyt-Bindungsstellen haben und in im Wesentlichen unmittelbarer Nähe zueinander angeordnet sind, so dass ein Schalter mit einem benachbarten Schalter wechselwirken kann.

26. Sehalterbaugruppe nach Anspruch 25, wobei die Schalter so geformt und angeordnet sind, dass der eine Schalter über eine Biomolekülbindung zu jeweiligen Biomolekülanalyt-Bindungsstellen desselben an einen benachbarten Schalter gekoppelt ist.

27. Schalter nach Anspruch 1, der einen bio-interaktiven "Transistor" zum Steuern der Übertragung eines Datenträgersignals umfasst, wobei die Vorrichtung Folgendes umfasst;
a) eine biologische Makromolekülstruktur,
b) wobei die Struktur wenigstens eine erste Konformation und eine zweite Konformation, die sich von der ersten Konformation unterscheidet, hat,
c) wobei die Struktur in einem Fluidgrenzflächenzustand an einem feststehenden Substrat verankert ist,
d) um so als Reaktion auf eine Steuersignaleingabe eine Umformung zwischen der ersten und der zweiten Konformation zu ermöglichen,
e) wobei ein erster und ein zweiter Teil der Makromolekülstruktur jeweilige wechselwirkende, erste und zweite Abschnitte eines trennungsempfindlichen Datenträgersignal-Übertragungsabschnitts der Transistorvorrichtung tragen,
f) wobei der erste und der zweite Teil der Makromolekülstruktur in der ersten und der zweiten Konformation der Struktur unterschiedliche Trennungsentfernungen haben, und
g) wobei in der ersten und der zweiten Konformation der Struktur unterschiedliche Ausgabesignale von dem Signalübertragungsabschnitt zu erhalten sind, wodurch unterschiedliche Steuerungssignaleingaben unterschiedliche Ausgabesignale liefern können.

## Revendications

1. Commutateur macromoléculaire destiné à être utilisé dans un dispositif d'acquisition et/ou de traitement de données, comprenant: une structure macromoléculaire comportant au moins un site de liaison d'ions, et au moins un site de liaison d'un analyte d'une biomolécule séparé formé et agencé de sorte à se lier à un analyte d'une biomolécule cible prédéterminé, ladite structure macromoléculaire pouvant être basculée entre plusieurs conformations discrètement différentes correspondant à différentes conditions de liaison d'ions au niveau du au moins un site de liaison d'ions, au moins en l'absence d'une quelconque autre liaison à ladite structure macromoléculaire, ladite structure macromoléculaire comportant en outre au moins un dispositif d'entrée électrochimique formé et agencé de sorte à appliquer de telles conditions de liaison d'ions différentes audit site de liaison d'ions en réponse à des signaux d'entrée externes correspondants,

2. Commutateur selon la revendication 1, dans lequel ledit au moins un dispositif d'entrée est formé et agencé de sorte à pouvoir fonctionner sous la commande de signaux d'entrée électriques externes.

3. Commutateur selon les revendications 1 ou 2, dans lequel ledit dispositif d'entrée électrochimique comprend un réservoir d'ions et un dispositif de commande du réservoir d'ions, formé et agencé de sorte à permettre au moins la capture d'ions dans ledit réservoir d'ions ou un dégagement d'ions de celui-ci.

4. Commutateur selon la revendication 3, dans lequel ledit dispositif de commande du réservoir d'ions comprend un système redox formé et agencé de sorte à permettre la capture d'ions dans ledit reservoir d'ions et le dégagement d'ions de celui-ci.

5. Commutateur selon l'une quelconque des revendications 1 à 4, dans lequel ledit au moins un dispositif d'entrée électrochimique est formé et agencé de sorte à entraîner une commutation réversible entre lesdites conditions de liaison d'ions différentes.

6. Commutateur selon la revendication 5, dans lequel sont agencés au moins deux dits sites de liaison d'un analyte d'une biomolécule.

7. Commutateur selon l'une quelconque des revendications précédentes, dans lequel au moins un dit site de liaison d'un analyte d'une biomolécule comporte une biomolécule compétitrice qui y est liée, ladite biomolécule compétitrice pouvant être substituée par ledit analyte de la biomolécule cible lors de l'utilisation du commutateur, par l'intermédiaire d'une liaison compétitrice.

8. Commutateur selon la revendication 7, dans lequel ladite biomolécule compétitrice est liée à ladite structure macromoléculaire de sorte à verrouiller ladite structure dans l'une desdites conformations contre un basculement vers une dite conformation différente.

9. Commutateur selon l'une quelconque des revendications 1 à 8, dans lequel ledit commutateur macromoléculaire comporte au moins un composant de dispositif de sortie d'un système de transmission de signaux de sortie, formé et agencé de sorte à servir dans la lecture de l'un des états suivants: l'état conformationnel de la structure macromoléculaire et la présence d'un analyte d'une biomolécule cible lié audit site de liaison de l'analyte de la biomolécule,

10. Commutateur selon la revendication 9, dans lequel ledit composant comprend une partie d'un dispositif de sortie sensible à une séparation.

11. Commutateur selon les revendications 9 ou 10, dans lequel ledit composant du dispositif de sortie comprend un marqueur fluorescent.

12. Commutateur selon la revendication 11, dans lequel ledit marqueur fluorescent se prête à une utilisation dans un système de transmission de signaux sensible à une séparation.

13. Commutateur selon la revendication 12, dans lequel ledit système de transmission, de signaux sensible à une séparation est un système de transfert d'énergie de résonance à fluorescence (FRET).

14. Commutateur selon l'une quelconque des revendications 9 à 13, dans lequel ladite structure macromoléculaire est accouplée à au moins un composant de traitement de signaux de données à semi-conducteurs.

15. Commutateur selon la revendication 14, dans lequel ledit au moins un composant de traitement de signaux de données à semi-conducteurs comprend un composant pour transformer un signal optique en un signal électronique.

16. Commutateur selon l'une quelconque des revendications 1 à 15, dans lequel ladite structure macromoléculaire est accouplée à au moins un circuit de traitement de données à semi-conducteurs, pour y transférer les données de manière pratiquement directe.

17. Commutateur selon l'une quelconque des revendications 1 à 16, dans lequel ladite structure macromoléculaire est retenue de manière captive à proximité dudit au moins un dispositif d'entrée.

18. Commutateur selon la revendication 17, dans lequel ladite structure macromoléculaire est ancrée sur un substrat solide.

19. Commutateur selon l'une quelconque des revendications 1 à 18, dans lequel ladite structure macromoléculaire comprend une structure de type de jonction de Holliday (HJ), dans laquelle au moins une partie des quatre structures de bras HJ est composée d'un matériau non polynucléotide, non incompatible avec le fonctionnement et l'application de la commutation de conformation de la structure macromoléculaire, et dans laquelle le point de ramification de la structure macromoléculaire de type HJ comporte au moins les quatre éléments à interaction de la séquence dimère polynucléoside d'un point de ramification HJ.

20. Conunutateur selon la revendication 19, dans lequel lesdits quatre éléments à interaction de la séquence dimère polynucléoside d'un point de ramification HJ comprennent les éléments suivants: TC, GT, AG et CA,

21. Commutateur selon l'une quelconque des revendications 1 à 18, dans lequel ladite structure macromoléculaire comprend au moins un polynucléotide.

22. Commutateur selon la revendication 21, dans lequel ladite structure macromoléculaire est une jonction de Holliday.

23. Commutateur selon l'une quelconque des revendications 1 à 18, dans lequel ladite structure macromoléculaire comprend au moins un polypeptide.

24. Assemblage de commutateur, comprenant plusieurs commutateurs selon l'une quelconque des revendications 1 à 23, comportant un dispositif d'entrée de signaux formé et agencé de sorte à appliquer des signaux d'entrée externes auxdits dispositifs d'entrée desdits commutateurs, d'une manière séparée dans le temps.

25. Assemblage de commutateur comprenant plusieurs commutateurs selon l'une quelconque des revendications 1 à 23, dans lequel lesdits commutateurs comportent des sites de liaison différents d'un analyte d'une biomolécule et sont agencés proximité pratiquement directe les uns des autres, de sorte qu'un dit commutateur peut coopérer avec un dit commutateur voisin.

26. Assemblage de commutateur selon la revendication 25, dans lequel lesdits commutateurs sont formés et agencés de sorte que ledit un commutateur est accouplé un dit commutateur voisin par une biomolécule se liant aux sites de liaison respectifs de l'analyte de la biomolécule.

27. Commutateur selon la revendication 1, comprenant un « transistors » à bio-interactivité pour contrôler la transmission d'un signal de support de données, ledit dispositif comprenant:
a) une structure macromoléculaire biologique;
b) ladite structure ayant au moins une première conformation et une deuxième conformation différente de ladite première conformation;
c) ladite structure étant ancrée sur un substrat fixe dans un état à jonction de fluide;
d) de sorte à permettre la transformation entre lesdites première et deuxième conformations en réponse à l'entrée d'un signal de commande;
e) des première et deuxième parties de ladite structure macromoléculaire, sur lesquelles sont montées des première et deuxième parties à interaction respectives d'une partie de transmission de signaux de support de données sensible à une séparation dudit dispositif de transistor:
f) lesdites première et deuxième parties de ladite structure macromoléculaire présentant des distances de séparation différentes dans lesdites première et deuxième conformations de la structure; et
g) dans lequel différents signaux de sortie peuvent être fournis par ladite partie de transmission de signaux dans lesdites première et deuxième conformations de ladite structure., des entrées de différents signaux de commande pouvant ainsi fournir différents signaux de sortie.
